(19) Europäisches Patentamt
European Patent Office
Office européen des brevets

(11) EP 4 775 964 A2

(12) **EUROPEAN PATENT APPLICATION**

(43) Date of publication:
**15.07.2026 Bulletin 2026/29**

(21) Application number: **26167202.6**

(22) Date of filing: **06.03.2018**

(51) International Patent Classification (IPC):
***G01N 21/64** (2006.01)*

(52) Cooperative Patent Classification (CPC):
(C-Sets available)
**C12Q 1/6874; G01N 21/6428;** G01N 2021/6421; G01N 2021/6441 (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.03.2017 US 201762468242 P**

(62) Document number(s) of the earlier application(s) in accordance with Art. 76 EPC:
**18712067.0 / 3 592 865**

(71) Applicants:
- **Illumina, Inc.**
  **San Diego, CA 92122 (US)**
- **Illumina Cambridge Limited**
  **Essex CB10 1XL (GB)**

(72) Inventors:
- **Liu, Xiaohai**
  **Essex CB10 1XL (GB)**
- **Vieceli, John**
  **San Diego CA 92122 (US)**
- **Langlois, Robert**
  **San Diego CA 92122 (US)**

(74) Representative: **Witte, Weller & Partner Patentanwälte mbB**
**Postfach 10 54 62**
**70047 Stuttgart (DE)**

Remarks:
•This application was filed on 24-03-2026 as a divisional application to the application mentioned under INID code 62.
•Claims filed after the date of filing of the application/after the date of receipt of the divisional application (Rule 68(4) EPC).

(54) **SINGLE LIGHT SOURCE, TWO-OPTICAL CHANNEL SEQUENCING**

(57) Disclosed is a system for determining the nucleotide sequence of polynucleotides. The system can comprise a light source, such as a laser or a LED, configured to generate light at a predetermined wavelength. A detector of the system can detect fluorescent emissions at a first wavelength and a second wavelength. A processor of the system identify the nucleotide as a first type if no fluorescent emission is detected by the at least one detector; identify the nucleotide as a second type if a fluorescent emission at the first wavelength of light is detected by the at least one detector; identify the nucleotide as a third type if a fluorescent emission at the second wavelength of light is detected by the at least one detector; and identify the nucleotide as a fourth type if fluorescent emissions at the first wavelength and the second wavelength of light are detected by the at least one detector.

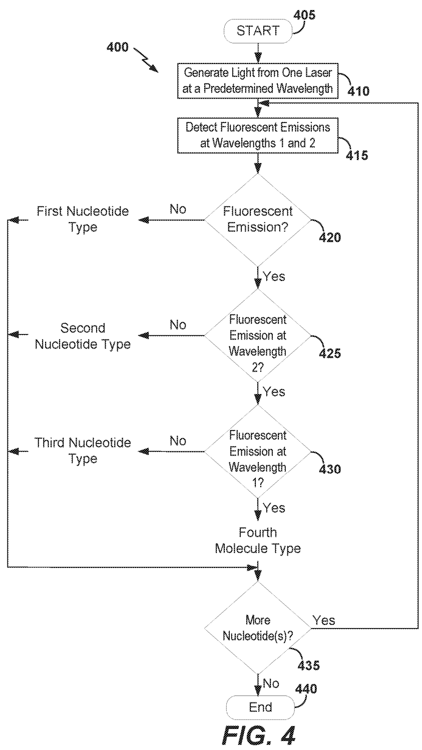

FIG. 4



Processed by Luminess, 75001 PARIS (FR)

(Cont. next page)

(52) Cooperative Patent Classification (CPC): (Cont.)

C-Sets
**C12Q 1/6874, C12Q 2537/165, C12Q 2563/107**

## Description

RELATED APPLICATIONS

The present application claims priority to U.S. Provisional Application No. 62/468242, filed on March 7, 2017. The content of this related application is incorporated herein by reference in its entirety.

BACKGROUND

Field

The present disclosure relates generally to the field of DNA sequencing, and more particularly relates to systems and methods for DNA sequencing utilizing a single light source and at least two dyes such as two fluorescent labels.

Description of the Related Art

Existing DNA sequencing systems and methods utilize two or more light sources to excite deoxyribonucleic acid analogs conjugated with fluorescent labels. However, in operation, light sources have high power consumptions and can generate a substantial amount of heat that needs to be dissipated. Fluorescent labels that can be efficiently excited by one light source can be subject to cross-talk whereby each label emits light at a wavelength that overlaps with other labels. When uncorrected, this cross-talk can make it difficult for DNA sequencing systems to properly call the correct nucleotide base during a sequencing run.

SUMMARY

Disclosed herein are systems and methods for determining the nucleotide sequence of polynucleotides. In one example, a system includes a single light source, such as a laser or a light-emitting diode, configured to generate light, such as light at a predetermined wavelength; at least one detector configured to detect fluorescent emissions off a fluorophore attached to a nucleotide, the at least one detector being configured to detect the fluorescent emissions at a first wavelength and a second wavelength; a processor configured to execute instructions that perform a method comprising: generating light from the light source onto a nucleotide; identifying the nucleotide as a first type when no fluorescent emission is detected by the at least one detector; identifying the nucleotide as a second type when a fluorescent emission at the first wavelength of light is detected by the at least one detector; identifying the nucleotide as a third type when a fluorescent emission at the second wavelength of light is detected by the at least one detector; and identifying the nucleotide as a fourth type when fluorescent emissions at the first wavelength and the second wavelength of light are detected by the at least one detector.

Another example is a computer-implemented method that includes generating light using a light source onto a fluorophore attached to a nucleotide; detecting fluorescent emissions off the fluorophore attached to the nucleotide at a first wavelength and a second wavelength using at least one detector; and identifying the nucleotide, comprising identifying the nucleotide as a first type when no fluorescent emission is detected by the at least one detector; identifying the nucleotide as a second type when a fluorescent emission at the first wavelength of light is detected by the at least one detector; identifying the nucleotide as a third type when a fluorescent emission at the second wavelength of light is detected by the at least one detector; and identifying the nucleotide as a fourth type when fluorescent emissions at the first wavelength and the second wavelength of light are detected by the at least one detector.

In another example, a system includes a single light source configured to generate light; at least one detector configured to detect four substantially different fluorescent emissions off different fluorophores attached to nucleotides; a processor configured to execute instructions that perform a method comprising: generating light from the light source onto a nucleotide; identifying the nucleotide as a first type when a first fluorescent emission is detected by the at least one detector; identifying the nucleotide as a second type when a second fluorescent emission is detected by the at least one detector; identifying the nucleotide as a third type when a third fluorescent emission is detected by the at least one detector; and identifying the nucleotide as a fourth type when a fourth fluorescent emission is detected by the at least one detector, wherein the first fluorescent emission, the second fluorescent emission, the third fluorescent emission, and the fourth fluorescent emissions have substantially different wavelengths.

BRIEF DESCRIPTION OF THE DRAWINGS

FIG. 1 is a schematic illustration showing an example single light source, two-optical channel sequencer.

FIG. 2 shows a functional block diagram of an example computer system for performing single light source, two-optical channel sequencing.

FIG. 3 is a flowchart of an example method for sequencing by synthesis utilizing single light source, two-optical channel sequencing.

FIG. 4 is a flowchart of an example method for performing base calling for single light source, two-optical channel sequencing.

FIG. 5 is a flowchart of an example method for performing single light source, two-optical channel sequencing.

FIG. 6 show outlines of nucleic acid clusters and their sequencing using single light source, two-optical channel sequencing.

FIGS. 7A-D are schematic plots showing color correction and phase correction for single light source, two-optical channel sequencing.

DETAILED DESCRIPTION

In the following detailed description, reference is made to the accompanying drawings, which form a part hereof. In the drawings, similar symbols typically identify similar components, unless context dictates otherwise. The illustrative embodiments described in the detailed description, drawings, and claims are not meant to be limiting. Other embodiments may be utilized, and other changes may be made, without departing from the spirit or scope of the subject matter presented herein. It will be readily understood that the aspects of the present disclosure, as generally described herein, and illustrated in the Figures, can be arranged, substituted, combined, separated, and designed in a wide variety of different configurations, all of which are explicitly contemplated herein.

Embodiments of the invention relate to next generation nucleotide sequencing systems that can identify all four nucleotide bases using a single light source and only two different optical channels. The sequencing systems can make use of a Sequencing by Synthesis process. During each sequencing cycle, four types of nucleotide analogs can be incorporated onto growing primers hybridized to polynucleotides being sequenced. In some embodiments, the four types of nucleotide analogs can include a deoxyguanosine triphosphate (dGTP) analog not conjugated with any fluorescent dye, a deoxythymidine triphosphate (dTTP) analog conjugated with a first fluorescent dye, a deoxycytidine triphosphate (dCTP) analog conjugated with a second fluorescent dye, and a deoxyadenosine triphosphate (dATP) analog conjugated with both the fluorescent dyes (or a mixture of two dATP analogs, one dATP analog with the first fluorescent dye and another dATP analog with the second fluorescent dye). The fluorescent dyes conjugated to the four types of nucleotide analogs are illustrative only, and not intended to be limiting. For example, the dTTP analog may not be conjugated with any fluorescent dye, the dCTP analog may be conjugated with a first fluorescent dye, the dATP analog may be conjugated with a second fluorescent dye, and the dGTP analog may be conjugated with both the fluorescent dyes (or a mixture of two dGTP analogs, one dGTP analog with the first fluorescent dye and another dGTP analog with the second fluorescent dye). As another example, the dCTP analog may not be conjugated with any fluorescent dye, the dATP analog may be conjugated with a first fluorescent dye, the dTTP analog may be conjugated with a second fluorescent dye, and the dGTP analog may be conjugated with both the fluorescent dyes (or a mixture of two dGTP analogs, one dGTP analog with the first fluorescent dye and another dGTP analog with the second fluorescent dye). As yet another example, the nucleotide analog not conjugated with any fluorescent dye may be dGTP, dTTP, dCTP, or dATP. The nucleotide analogy conjugated with the first fluorescent dye or the second fluorescent dye may be dGTP, dTTP, dCTP, or dATP. The nucleotide analog conjugated with two fluorescent dyes may be dGTP, dTTP, dCTP, or dATP. The dGTP, dTTP, dCTP, or dATP analog can comprise a mixture of two analogs, one analog with the first fluorescent dye and another analog with the second fluorescent dye.

The light source (e.g., a laser or a light-emitting diode) can excite the two fluorescent dyes. The first fluorescent dye fluoresces at a first wavelength and can be captured in a first fluorescent image. The second fluorescent dye fluoresces at a second wavelength and can be captured in a second fluorescent image. Intensities of the fluorescent emissions captured are extracted from the two fluorescent images. In some embodiments, the two fluorescent dyes may be subject to cross-talk, and the fluorescent emissions of the dTTP analog and the dCTP analog can be captured in both the fluorescent images. Thus, the extracted intensities need to be corrected by, for example, color correction. In some embodiments, the two fluorescent dyes may have a large stokes shift, and the fluorescent emissions may have minimal, or no, cross-talk.

In some embodiments, the one of the two fluorescent dyes can be a normal stokes shift dye and the other of the fluorescent dyes can be a long stokes shift dye. Non-limiting examples of a normal stokes shift dye include Alexa 488 or its dye analogues (such as 3,6-Bis(ethylamino)-2,7-dimethyl-[2-carboxylato-5-(3-carboxypropyloxy)phenyl]xanthylium betaine (dye I-3), and 3,6-Bis(ethylamino)-2,7-dimethyl-[2-carboxylato-4-(3-carboxypropyloxy)phenyl]xanthylium betaine (dye I-4) disclosed in US Patent No. 8,754,244, the content of which is incorporated herein in its entirety). The normal stokes shift dye can be excited with a laser or a light-emitting diode (LED) light source with a wavelength of 488 nm and can

have an emission peak at 520 nm. A long stokes shift dye can be dye NR520LS in PCT Patent Application No. PCT/GB2016/051474, the content of which is incorporated herein in its entirety). The long stokes shift dye can have an emission peak at 590 nm. In some embodiments, the two fluorescent dyes can be Cy3 (with emission peak at around 575nm) and a fluorescence resonance energy transfer (FRET) pair dye Cy3-Cy5 (with emission peak at 670nm).

Color correction can utilize a color matrix to condition the extracted intensities utilizing properties of the underlying distribution of intensities within each fluorescent image. The color matrix can be estimated by plotting the extracted intensities from the first fluorescent image versus the extracted intensities from corresponding positions in the second fluorescent image at positions ($x_i$, $y_i$). $x_i$ and $y_i$ denote the extracted intensity from a position i of growing primer-polynucleotides in the second fluorescent image and the first fluorescent image respectively. The plotted intensities at positions ($x_i$, $y_i$) are converted to polar coordinates ($r_i$, $\theta_i$), and a radius-weighted histogram of angles $\theta_i$ is computed. The two local maxima, $\theta_1$ and $\theta_2$, in the radius-weighted histogram can be used to estimate the color matrix. The color matrix can be $\begin{pmatrix} 1 & \tan(\theta_1) \\ \tan(90-\theta_2) & 1 \end{pmatrix}$.

After applying the inverse of the color matrix to the plotted intensities at positions ($x_i$, $y_i$), the bases of nucleotides incorporated can be determined. For example, if no fluorescent emission is detected, the nucleotide incorporated can be the dGTP analog. If fluorescent emission is detected in the second fluorescent image and not the first fluorescent image, the nucleotide incorporated can be the dTTP analog. If fluorescent emission is detected in the first fluorescent image and not the first fluorescent image, the nucleotide incorporated can be the dCTP analog. If fluorescent emissions are detected in both fluorescent images, the nucleotide incorporated can be the dATP analog.

## Definitions

Unless defined otherwise, technical and scientific terms used herein have the same meaning as commonly understood by one of ordinary skill in the art to which the present disclosure belongs. *See, e.g.* Singleton et al., Dictionary of Microbiology and Molecular Biology 2nd ed., J. Wiley & Sons (New York, NY 1994); Sambrook et al., Molecular Cloning, A Laboratory Manual, Cold Spring Harbor Press (Cold Spring Harbor, NY 1989). For purposes of the present disclosure, the following terms are defined below.

## Single-Light Source, Two-Optical Channel Sequencer

Disclosed herein are systems and methods for determining the nucleotide sequence of polynucleotides using a single light source (e.g., a laser or a LED). In one embodiment, there are at least two dyes used to sequence a polynucleotide. FIG. 1 is a schematic illustration showing an example single light source, two-optical channel sequencing system 100. The single light source, two-optical channel sequencing system 100 can be configured to utilize sequencing methods based on two dyes, for example, a first fluorescent label and a second fluorescent label. Non-limiting examples of the sequencing methods utilized can include sequencing by synthesis and Heliscope single molecule sequencing. The single light source, two-optical channel sequencing system 100 can include an optics system 102 configured to generate raw sequencing data using sequencing reagents supplied by a fluidics system 104 that is part of the single light source, two-optical channel sequencing system 100. The raw sequencing data can include fluorescent images captured by the optics system 102. A computer system 106 that is part of the single light source, two-optical channel sequencing system 100 can be configured to control the optics system 102 and the fluidics system 104 via communication channels 108A and 108B. For example, a computer interface 110 of the optics system 102 can be configured to communicate with the computer system 106 through the communication channel 108A.

During sequencing reactions, the fluidics system 104 can direct the flow of reagents through one or more reagent tubes 112 to and from a flowcell 114 positioned on a mounting stage 116. The reagents can be, for example, fluorescently labeled nucleotides, buffers, enzymes, and cleavage reagents. The flowcell 114 can include at least one fluidic channel. The flowcell 114 can be a patterned array flowcell or a random array flowcell. The flowcell 114 can include multiple clusters of single-stranded polynucleotides to be sequenced in the at least one fluidic channel. The lengths of the polynucleotides can vary ranging, for example, from 200 bases to 1000 bases. The polynucleotides can be attached to the one or more fluidic channels of the flowcell 114. In some embodiments, the flowcell 114 can include a plurality of beads, wherein each bead can include multiple copies of a polynucleotide to be sequenced. The mounting stage 116 can be configured to allow proper alignment and movement of the flowcell 114 in relation to the other components of the optics system 102. In one embodiment, the mounting stage 116 can be used to align the flowcell 114 with a lens 118.

The optics system 102 can include a single light source 120, such as a single laser or a single LED source, configured to generate light at a predetermined wavelength, for example 532 nm. The light generated by the light source 120 can pass through a fiber optic cable 122 to excite fluorescent labels in the flowcell 114. The lens 118, mounted on a focuser 124, can move along the z-axis. The focused fluorescent emissions can be detected by a detector 126, for example

a charge-coupled device (CCD) sensor or a complementary metal oxide semiconductor (CMOS) sensor.

A filter assembly 128 of the optics system 102 can be configured to filter the fluorescent emissions of the fluorescent labels in the flowcell 114. The filter assembly 128 can include a first filter and a second filter. Each filter can be a longpass filter, a shortpass filter, or a bandpass filter, depending on the types of fluorescent molecules being used in the system. The first filter can be configured to detect the fluorescent emissions of the first fluorescent labels by the detector 126. The second filter can be configured to detect the fluorescent emissions of the second fluorescent labels by the detector 126. With two filters in the filter assembly 128, the detector 126 can detect two different wavelengths of light. The two wavelengths of light can be from the same fluorescent label or different fluorescent labels. The two wavelengths of light can be, for example, at least 20 nm apart.

In some embodiments, the optics system 102 can include a dichroic configured to split the fluorescent emissions. The optics system 102 can include two detectors, a first detector coupled with a first filter for detecting fluorescent emissions at a first wavelength and a second detector coupled with a second filter for detecting fluorescent emissions at a second wavelength. After splitting the fluorescent emissions with a dichroic, the optics system 102 can detect fluorescent emissions simultaneously (or close in time) at two wavelengths using the two detectors coupled with different filters. This configuration can speed up the imaging process. Accordingly, multiple flowcells can be processed simultaneously, with one flowcell undergoing imaging while nucleotide analogs are incorporated into polynucleotide clusters of the one or more other flowcells.

In use, a sample having a polynucleotide to be sequenced is loaded into the flowcell 114 and placed in the mounting stage 116. The computer system 106 then activates the fluidics system 104 to begin a sequencing cycle. During sequencing reactions, the computer system 106 instructs the fluidics system 104, through the communication interface 108B, to supply reagents, for example nucleotide analogs, to the flowcell 114. Through the communication interface 108A and the computer interface 110, the computer system 106 is configured to control the light source 120 of the optics system 102 to generate light at a predetermined wavelength and shine onto nucleotide analogs incorporated into growing primers hybridized to polynucleotides being sequenced. The computer system 106 controls the detector 126 of the optics system 102 to capture the emission spectra of the nucleotide analogs in fluorescent images. The computer system 106 receives the fluorescent images from the detector 126 and process the fluorescent images received to determine the nucleotide sequence of the polynucleotides being sequenced.

Light Source and Filters

The single light source, two-optical channel sequencing system 100 can utilize one light source, such as a laser or a LED, capable of exciting two fluorescent labels with emission spectra that are sufficiently non-overlapping. The wavelength of the light generated by the light source 120 can vary, for example, ranging from 400 nm to 800 nm. In some embodiments, the wavelength of the light generated by the light source 120 can be, or be about, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800 nm, or a number or a range between any two of these values. In some embodiments, the wavelength of the light generated by the light source 120 can be at least, or at most, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, or 800 nm.

The detector 126, with the filter assembly 128, can be configured to detect light of, or about, two different wavelengths, for example a first wavelength and a second wavelength. The first wavelength and the second wavelength can be apart from each other, for example, ranging from 10 nm to 100 nm. In some embodiments, the first wavelength and the second wavelength can be, or be about, 10, 11, 12, 13, 14, 15, 16, 17, 18, 19, 20, 30, 40, 50, 60, 70, 80, 90, 100 nm, or a number or a range between any two of these values, apart. In some embodiments, the first wavelength and the second wavelength can be at least, or at most, 10, 11, 12, 13, 14, 15, 16, 17, 18, 19, 20, 30, 40, 50, 60, 70, 80, 90, or 100 nm apart.

The number of filters in the filter assembly 128 can vary, ranging from 1 to 10. In some embodiments, the number of filters in the filter assembly 128 can be, or be about, 1, 2, 3, 4, 5, 6, 7, 8, 9, 10, or a range between any two of these values. In some embodiments, the number of filters in the filter assembly 128 can be at least, or at most, 1, 2, 3, 4, 5, 6, 7, 8, 9, or 10.

A filter can be a bandpass filter and can have peak transmittance of varying wavelength, ranging from 400 nm to 800 nm. In some embodiments, the peak transmittance can be, or be about, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800 nm, or a number or a range between any two of these values. In some embodiments, the peak transmittance can be at least, or at most, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, or 800 nm. The width of the filter can vary, for example, ranging from 1 nm to 50 nm. In some embodiments, the width of the filter can be, or be about, 1, 2, 3, 4, 5, 6, 7, 8, 9, 10, 20, 30, 40, 50 nm, or a number or a range between any two of these values. In some embodiments, the width of the filter can be at least, or at most, 1, 2, 3, 4, 5, 6, 7, 8, 9, 10, 20, 30, 40, or 50 nm.

Fluorescent Labels

The fluorescent labels utilized by the systems and methods disclosed herein can have different peak absorption wavelengths, for example, ranging from 400 nm to 800 nm. In some embodiments, the peak absorption wavelengths of the fluorescent labels can be, or be about, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800 nm, or a number or a range between any two of these values. In some embodiments the peak absorption wavelengths of the fluorescent labels can be at least, or at most, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, or 800 nm.

The fluorescent labels can have different peak emission wavelength, for example, ranging from 400 nm to 800 nm. In some embodiments, the peak emission wavelengths of the fluorescent labels can be, or be about, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800 nm, or a number or a range between any two of these values. In some embodiments the peak emission wavelengths of the fluorescent labels can be at least, or at most, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, or 800 nm.

The fluorescent labels can have different stokes shift, for example, ranging from 10 nm to 200 nm. In some embodiments, the stoke shift can be, or be about, 10, 20, 30, 40, 50, 60, 70, 80, 90, 100, 110, 120, 130, 140, 150, 160, 170, 180, 190, 200 nm, or a number or a range between any two of these values. In some embodiments, the stoke shift can be at least, or at most, 10, 20, 30, 40, 50, 60, 70, 80, 90, 100, 110, 120, 130, 140, 150, 160, 170, 180, 190, or 200 nm.

The systems and methods disclosed herein can utilize two fluorescent labels, for example a first fluorescent label and a second fluorescent label, can have overlapping emission spectra and can be subject to cross-talk. In some embodiments, the peak emission wavelengths of the two fluorescent labels can vary, for example, ranging from 10 nm to 200 nm. In some embodiments, the peak emission wavelengths of the two fluorescent labels can be, or be about, 10, 20, 30, 40, 50, 60, 70, 80, 90, 100, 110, 120, 130, 140, 150, 160, 170, 180, 190, 200 nm, or a number or a range between any two of these values. In some embodiments, the peak emission wavelengths of the two fluorescent labels can be at least, or at most, 10, 20, 30, 40, 50, 60, 70, 80, 90, 100, 110, 120, 130, 140, 150, 160, 170, 180, 190, or 200 nm. The detector 126, with one of the filters in the filter assembly 128, can detect fluorescent emissions of the first fluorescent label. The detector 126, with another filter in the filter assembly 128, can detect fluorescent emissions of the second fluorescent label.

In some embodiments, the one of the two fluorescent dyes can be a normal stokes shift dye and the other of the fluorescent dyes can be a long stokes shift dye. Non-limiting examples of a normal stokes shift dye include Alexa 488 or its dye analogues (such as 3,6-Bis(ethylamino)-2,7-dimethyl-[2-carboxylato-5-(3-carboxypropyloxy)phenyl]xanthylium betaine (dye I-3), and 3,6-Bis(ethylamino)-2,7-dimethyl-[2-carboxylato-4-(3-carboxypropyloxy)phenyl]xanthylium betaine (dye I-4) in US Patent No. 8,754,244). The normal stokes shift dye can be excited with a laser or a LED light source with a wavelength of 488 nm and can have an emission peak at 520 nm. A long stokes shift dye can be dye NR520LS in PCT Patent Application No. PCT/GB2016/051474. The long stokes shift dye can have an emission peak at 590 nm. In some embodiments, the two fluorescent dyes can be Cy3 (with emission peak at around 575nm) and a fluorescence resonance energy transfer (FRET) pair dye Cy3-Cy5 (with emission peak at 670nm).

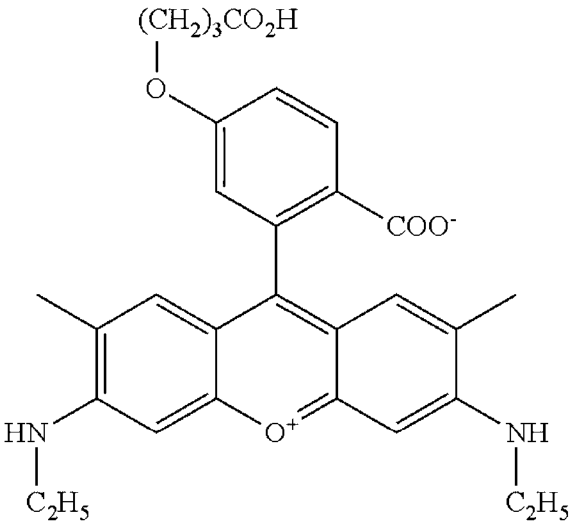

I-3

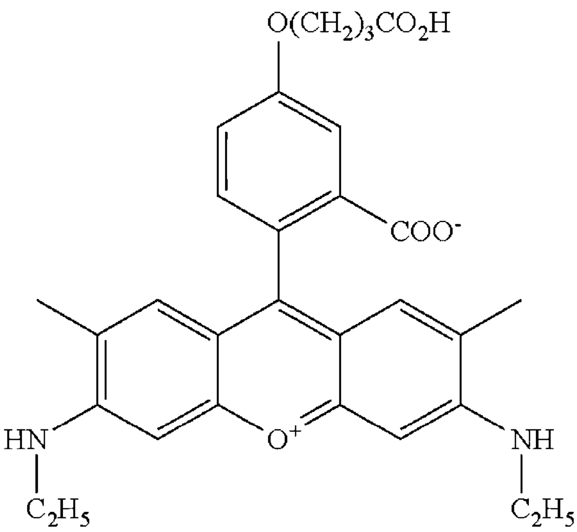

I-4

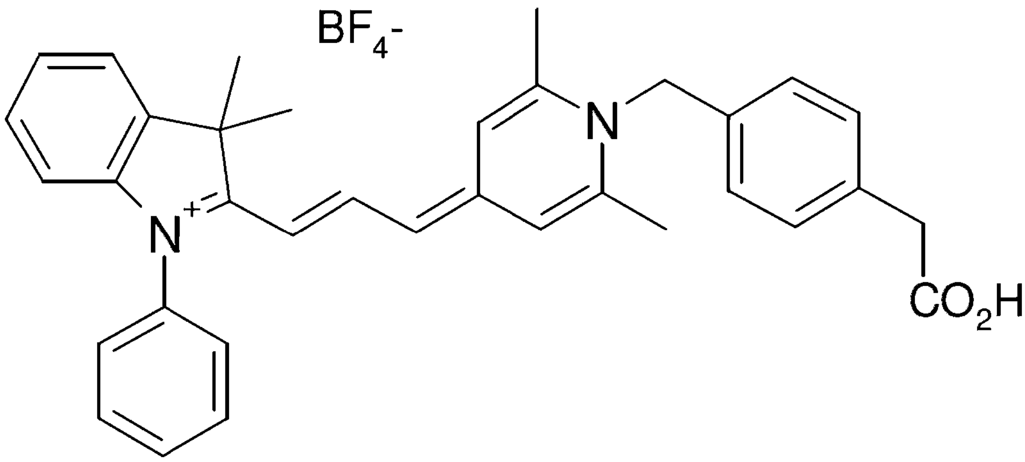

NR520LS

Computer System

The computer system 106 of the single light source, two-optical channel sequencing system 100 can be configured to control the optics system 102 and the fluidics system 104 as discussed above. While many configurations are possible for the computer system 106, one embodiment is illustrated in FIG. 2. As shown in FIG. 2, the computer system 106 can include a processor 202 that is in electrical communication with a memory 204, a storage 206, and a communication interface 208.

The processor 202 can be configured to execute instructions that cause the fluidics system 104 to supply reagents to the flowcell 114 during sequencing reactions. The processor 202 can execute instructions that control the light source 120 of the optics system 102 to generate light at a predetermined wavelength. The processor 202 can execute instructions that control the detector 126 of the optics system 102 and receive data from the detector 126. The processor 202 can execute instructions to process data, for example fluorescent images, received from the detector 126 and to determine the nucleotide sequence of polynucleotides based on the data received form the detector 126.

The memory 204 can be configured to store instructions for configuring the processor 202 to perform the functions of the computer system 106 when the single light source, two-optical channel sequencing system 100 is powered on. When the single light source, two-optical channel sequencing system 100 is powered off, the storage 206 can store the instructions for configuring the processor 202 to perform the functions of the computer system 106. The communication interface 208 can be configured to facilitate the communications between the computer system 106, the optics system 102, and the fluidics system 104.

The computer system 106 can include a user interface 210 configured to communicate with a display device (not shown) for displaying the sequencing results of the single light source, two-optical channel sequencing system 100. The

user interface 210 can be configured to receive inputs from users of the single light source, two-optical channel sequencing system 100. An optics system interface 212 and a fluidics system interface 214 of the computer system 106 can be configured to control the optics system 102 and the fluidics system 104 through the communication links 108A and 108B illustrated in FIG. 1. For example, the optics system interface 212 can communicate with the computer interface 110 of the optics system 102 through the communication link 108A.

The computer system 106 can include a nucleic base determiner 216 configured to determine the nucleotide sequence of polynucleotides using the data received from the detector 126. The nucleic base determiner 216 can include one or more of: a template generator 218, a location registrator 220, an intensity extractor 222, an intensity corrector 224, a base caller 226, and a quality score determiner 228. The template generator 218 can be configured to generate a template of the locations of polynucleotide clusters in the flowcell 114 using the fluorescent images captured by the detector 126. The location registrator 220 can be configured to register the locations of polynucleotide clusters in the flowcell 114 in the fluorescent images captured by the detector 126 based on the location template generated by the template generator 218. The intensity extractor 222 can be configured to extract intensities of the fluorescent emissions from the fluorescent images to generate extracted intensities. The intensity corrector 224 can be configured to reduce or eliminate the cross-talk between the fluorescent labels by, for example, color correcting the extracted intensities to generate corrected intensities. In some embodiments, the intensity corrector 224 can phase correct or prephase correct extracted intensities. The base caller 226 can be configured to determine the bases of the polynucleotide from the corrected intensities. The bases of the polynucleotides determined by the base caller 226 can be associated with quality scores determined by the quality score determiner 228.

Sequencing by Synthesis

FIG. 3 is a flowchart of an example method 300 for sequencing by synthesis utilizing the sequencing system 100. After the method 300 begins at block 305, a flowcell 114 including fragmented polynucleotide fragments (e.g., fragmented single- or double-stranded polynucleotide fragments) is received at block 310. The fragmented polynucleotide fragments can be generated from a deoxyribonucleic acid (DNA) sample. The DNA sample can be from various sources for example, a biological sample, a cell sample, an environmental sample, or any combination thereof. The DNA sample can include one or more of a biological fluid, a tissue, and cells from a patient. For example, the DNA sample can be taken from, or include, blood, urine, cerebrospinal fluid, pleural fluid, amniotic fluid, semen, saliva, bone marrow, a biopsy sample, or any combination thereof.

The DNA sample can include DNA from cells of interest. The cells of interest can vary and in some embodiments express a malignant phenotype. In some embodiments, the cells of interest can include tumor cells bone marrow cells, cancer cells, stem cells endothelial cells, virally infected cells pathogenic, parasitic organism cells or any combination thereof.

The lengths of fragmented polynucleotide fragments can range from 200 bases to 1000 bases. Once the flowcell 114 including fragmented polynucleotide fragments are received at block 310, the process 300 moves to block 320 where the polynucleotide fragments are bridge-amplified into clusters of polynucleotide fragments attached to the inside surface of one or more channels of a flowcell, for example the flowcell 114. The inside surface of the one or more channels of the flowcell can include two types of primers, for example a first primer type (P1) and a second primer type (P2) and the DNA fragments can be amplified by well-known methods.

After generating clusters within the flowcell 114, the process 300 can begin a Sequencing by Synthesis process. The Sequencing by Synthesis process can include determining the nucleotide sequence of clusters of single-stranded polynucleotide fragments. To determine the sequence of a cluster of single-stranded polynucleotide fragments with the sequence 5'-P1-F-A2R-3', primers with the sequence A2F, which are complementary of the sequence A2R, can be added and extended at block 325 with nucleotide analogs with zero, one, or two labels by a DNA polymerase to form growing primer-polynucleotides.

During each sequencing cycle, four types of nucleotide analogs can be added and incorporated onto the growing primer-polynucleotides. The four types of nucleotide analogs can have different modifications. For example, the first type of nucleotide can be an analog of deoxyguanosine triphosphate (dGTP) not conjugated with any fluorescent label. The second type of nucleotide can be an analog of deoxythymidine triphosphate (dTTP) conjugated with the first type of fluorescent label via a linker. The third type of nucleotide can be an analog of deoxycytidine triphosphate (dCTP) conjugated with the second type fluorescent label via a linker. The fourth type of nucleotide can be an analog of deoxyadenosine triphosphate (dATP) conjugated with both the first type of fluorescent label and the second type of fluorescent label via one or more linkers. The linkers may include one or more cleavage groups. Prior to the subsequent sequencing cycle, the fluorescent labels can be removed from the nucleotide analogs. For example, a linker attaching a fluorescent label to a nucleotide analog can include an azide and/or an alkoxy group, for example on the same carbon, such that the linker may be cleaved after each incorporation cycle by a phosphine reagent, thereby releasing the fluorescent label from subsequent sequencing cycles.

The nucleotide triphosphates can be reversibly blocked at the 3' position so that sequencing is controlled and no more than a single nucleotide analog can be added onto each extending primer-polynucleotide in each cycle. For example, the 3' ribose position of a nucleotide analog can include both alkoxy and azido functionalities which can be removable by cleavage with a phosphine reagent, thereby creating a nucleotide that can be further extended. After the incorporation of nucleotide analogs, the fluidics system 104 can wash the one or more channels of the flowcell 114 in order to remove any unincorporated nucleoside analogs and enzyme. Prior to the subsequent sequencing cycle, the reversible 3' blocks can be removed so that another nucleotide analog can be added onto each extending primer-polynucleotide.

At block 330, a single light source such as the laser 120 or an LED source can excite the two fluorescent labels at a predetermined wavelength. In some embodiments, the single laser or the LED source may be non-tunable. At block 335, signals from the fluorescent labels can be detected. Detecting the fluorescent labels can include capturing fluorescent emissions in two fluorescent images at a first wavelength and a second wavelength by, for example, the detector 126 using two filters. The fluorescent emissions of the first fluorescent label can be at, or around, the first wavelength, and the fluorescent emissions of the second fluorescent label can be at, or around, the second wavelength. The fluorescent images can be stored for later processing offline. In some embodiments, the fluorescent images can be processed to determine the sequence of the growing primer-polynucleotides in each cluster in real time.

A determination can be made at decision block 340 whether to detect more nucleotides based on, for example, the quality of the signal or after a predetermined number of bases. If more nucleotides are to be detected, then nucleotide determination of the next sequencing cycle can be performed starting again at block 325 with nucleotide analogs with zero, one, or two labels added to extend the primer-polynucleotides. Prior to the next sequencing cycle, the fluorescent labels can be removed from the incorporated nucleotide analogs, and the reversible 3' blocks can be removed so that another nucleotide analog can be added onto each extending primer-polynucleotide.

In offline fluorescent imaging processing, if there is no additional nucleotide to be detected at decision block 340, the fluorescent images comprising the fluorescent signals detected can be processed at block 345, and the bases of the nucleotides incorporated can be determined. For each nucleotide base determined, a quality score can be determined at block 350. After all the fluorescent images are processed, the process 300 can terminate at block 355.

## Base Calling

Base calling has been described in US Patent No. 8,965,076, the content of which is incorporated herein in its entirety. Briefly, base calling can refer to the process of determining bases of the nucleotides incorporated into the clusters of growing primer-polynucleotides being sequenced to be guanine (G), thymine (T), cytosine (C), or adenine (A). FIG. 4 is a flowchart of an example method 400 for performing base calling utilizing the sequencing system 100. Processing detected signals at block 345 illustrated in FIG. 3 can include performing base calling of the method 400. After beginning at block 405, light of a predetermined wavelength can be generated using a light source and can shine onto nucleotide analogs at block 410. For example, the computer system 106, through its optics system interface 212 and the communication channel 108A, can cause the light source 120 to generate light at the predetermined wavelength.

The light source-generated light can shine onto nucleotide analogs incorporated into growing primer-polynucleotides attached on inside surface of one or more channels of a flow cell, for example, the flowcell 114. The primer-polynucleotides can include clusters of single-stranded polynucleotide fragments hybridized to sequencing primers. The nucleotide analogs each can include zero, one, or two fluorescent labels. The two fluorescent labels can be a first fluorescent label and a second fluorescent label. The fluorescent labels, after being excited by the light source-generated light, can emit fluorescent emissions. For example, the first fluorescent label can produce fluorescent emissions at the first wavelength which can be captured in, for example, a first fluorescent image. The second fluorescent label can produce fluorescent emissions at the second wavelength which can be captured in, for example, a second fluorescent image.

The nucleotide analogs can include a first type of nucleotide, a second type of nucleotide, a third type of nucleotide, and a fourth type of nucleotide. The first type of nucleotide, for example an analog of deoxyguanosine triphosphate (dGTP), is not conjugated to the first fluorescent label or the second fluorescent label. The second type of nucleotide, for example an analog of deoxythymidine triphosphate (dTTP), can be conjugated with the first type of fluorescent label, and not the second type of fluorescent label. The third type of nucleotide, for example an analog of deoxycytidine triphosphate (dCTP), can be conjugated with the second type fluorescent label, and not the first type of fluorescent label. The fourth type of nucleotide, for example an analog of deoxyadenosine triphosphate (dATP), can be conjugated with both the first type of fluorescent label and the second type of fluorescent label.

At block 415, fluorescent emissions of the nucleotide analogs at the first wavelength and the second wavelength can be detected using at least one detector. For example, the detector 126 can capture two fluorescent images, a first fluorescent image at the first wavelength and a second fluorescent image at the second wavelength. After receiving the two fluorescent images from the optics system 102, the nucleic base determiner 216 can determine the presence or the absence of fluorescent emissions in the two fluorescent images.

Because the first type of nucleotide is not conjugated to the first fluorescent label or the second fluorescent label,

the first type of nucleotide can produce no, or minimal, fluorescent emission at the first wavelength or at the second wavelength. At decision block 420, if no fluorescent emission is detected, the nucleotide can be determined to be the first type of nucleotide, for example dGTP. If any or more than minimal fluorescent emission is detected, the method 400 can proceed to decision block 425.

Because the second type of nucleotide is conjugated with the first type of fluorescent label, and not the second type of fluorescent label, the second type of nucleotide can produce fluorescent emissions at the first wavelength and no, or minimal, fluorescent emission at the second wavelength. At decision block 425, if no fluorescent emission at the second wavelength is detected in the second fluorescent image, and from decision block 420, fluorescent emissions at the first wavelength are detected in the first fluorescent image, then the nucleotide can be determined to be the second type of nucleotide, for example dTTP. If fluorescent emissions are detected at the second wavelength, the method 400 can proceed to decision block 430.

Because the third type of nucleotide is conjugated with the second type fluorescent label, and not the first type of fluorescent label, the third type of nucleotide can produce fluorescent emissions at the second wavelength and no, or minimal, fluorescent emission at the first wavelength. At decision block 430, if no fluorescent emission at the first wavelength is detected in the first fluorescent image, and from decision block 425, fluorescent emissions at the second wavelength are detected in the second fluorescent image, then the nucleotide can be determined to be the third type of nucleotide, for example dCTP.

Because the fourth type of nucleotide is conjugated with both the first type of fluorescent label and the second type of fluorescent label, the fourth type of nucleotide can produce fluorescent emissions at the first wavelength or the second wavelength. At decision block 430, if fluorescent emissions are detected at the first wavelength in the first fluorescent image, and from decision block 425, fluorescent emissions can be detected at the second wavelength in the second fluorescent image, then the nucleotide can be determined to be the fourth type of nucleotide, for example dATP.

The flowcell 114 can include clusters of growing primer-polynucleotides to be sequenced. At decision block 435, if there is at least one more cluster with fluorescent emissions to be processed for a given sequencing cycle, the method 400 can continue at block 415. If no more cluster of single-stranded polynucleotide is to be processed, the method 400 can end at block 440.

Workflow for Single Light Source, Two-optical channel Sequencing

*Cycle 1: Template Generation, Location Registration, and Intensity Extraction*

FIG. 5 is a flowchart of an example method 500 for performing single light source, two-optical channel sequencing. The single light source, two-optical channel sequencing system 100 can perform the method 500. After beginning at block 505, a light source can generate light at a predetermined wavelength onto nucleotides at block 510. At block 515, the fluorescent emissions from a first fluorescent label at a first wavelength and from a second fluorescent label at a second wavelength can be detected using, for example, at least one detector to generate a first fluorescent image and a second fluorescent image. Detecting fluorescent emissions can include determining the intensities of fluorescent emissions. After receiving the two fluorescent images, a location template can be generated at block 520 by, for example, the template generator 218.

Generating a location template may be necessary during the first sequencing cycle to determine the locations of the clusters of single-stranded polynucleotides. FIG. 6 show outlines of nucleic acid clusters and their sequencing using single light source, two-optical channel sequencing. During the first sequencing cycle, the locations of the clusters are unknown. A flowcell can include four clusters, clusters 1-4. During the first sequencing cycle, the template generator 218 can determine the existence of the clusters 1, 2, and 4 in the flowcell.

During the first sequencing cycle, a first fluorescent image 602 and a second fluorescent image 604 of a flowcell at a first state 606, corresponding to the first sequencing cycle, can be generated. The nucleotide analogs incorporated into the clusters of growing primer-polynucleotides can vary. For example, the nucleotide incorporated into the cluster 1 can be an analog of deoxyadenosine triphosphate (dATP) conjugated with both the first type of fluorescent label and the second type of fluorescent label. The first fluorescent image 602 can capture the fluorescent emissions of the first type of fluorescent label on the dATP analog. The second fluorescent image 604 can capture the fluorescent emissions of the second type of fluorescent label on the dATP analog. The template generator 218 can determine from the first fluorescent image 602 or the second fluorescent image 604 the existence of the cluster 1 at the particular cluster 1 location.

The nucleotide incorporated into the cluster 2 can be an analog of deoxycytidine triphosphate (dCTP) conjugated with the second type fluorescent label, and not the first type of fluorescent label. The second fluorescent image can capture the fluorescent emissions of the second type of fluorescent label on the dCTP analog. If the first fluorescent label and the second fluorescent label are subject to cross-talk, the cluster 2 can have some fluorescent emissions on the first fluorescent image. The template generator 218 can determine from the second fluorescent image 604 the existence of the cluster 2 at the particular cluster 2 location.

The nucleotide incorporated into the cluster 3 can be an analog of deoxyguanosine triphosphate (dGTP) not conjugated to the first fluorescent label or the second fluorescent label. The first fluorescent image 602 and the second fluorescent image 604 thus have no, or minimal, fluorescent emission from the cluster 3. The template generator 218 may be unable to determine from the first fluorescent image 602 and the second fluorescent image 604 the existence of the cluster 3 at the particular cluster 3 location.

The nucleotide incorporated into the cluster 4 can be an analog of deoxythymidine triphosphate (dTTP) conjugated with the first type of fluorescent label, and not the second type of fluorescent label. The first fluorescent image 602 can capture the fluorescent emissions of the first type of fluorescent label on the dTTP analog. If the first fluorescent label and the second fluorescent label are subject to cross-talk, the cluster 4 can have some fluorescent emissions on the second fluorescent image 604. The template generator 218 can determine from the first fluorescent image the existence of the cluster 4 at the particular cluster 4 location.

The template generator 218 can generate a location template of the clusters 1, 2, and 4 based on the first fluorescent image 602 and the second fluorescent image 604 in the first sequencing cycle. In some embodiments, generating the location template can include detecting cross-talk between the first fluorescent label and the second fluorescent label. The cross-talk can advantageously make image registration more robust, especially in the lowdiversity context because the emissions of the fluorescent labels can be captured in both the first fluorescent image 602 and the second fluorescent image 604.

*Cycle 2: Template Generation and Location Registration*

Generating a location template may be necessary during the second sequencing cycle, when random flowcells are used, to determine the locations of the clusters of single-stranded polynucleotides. After the first sequencing cycle, the locations of the cluster 3 can be unknown. The nucleotide incorporated into the cluster 3 during the second sequencing cycle can be an analog of deoxycytidine triphosphate (dTTP) conjugated with the first type fluorescent label, and not the second type of fluorescent label. A first fluorescent image 612 can capture the fluorescent emissions of the first type of fluorescent label on the dTTP analog. During the second sequencing cycle, the template generator 218 can determine from the first fluorescent image 612 the existence of the cluster 3 at the particular cluster 3 location. Template generation when patterned flowcells are used has been described in US Patent Application No. 14/530,299, the content of which is incorporated herein in its entirety.

*Location Registration and Intensity Extraction*

Referring to FIG. 5, at block 525, the cluster locations in the location template can be registered to the fluorescent images captured for the first sequencing cycle and the subsequent sequencing cycles. The fluorescent intensities of the clusters of growing primer-polynucleotides at the registered locations, for example the locations 1, 2, and 4, can be extracted at block 530. The extracted intensities can be corrected at 535 to generate corrected intensities. Correcting extracted intensities by, for example, the intensity corrector 224 can include one or more of spatial normalization at block 540, color correction at block 545, or phasing correction at block 550.

*Spatial Normalization, Color Correction, and Phasing Correction*

Spatial normalization can include normalizing the intensities of fluorescent emissions in different fluorescent images of a sequencing cycle to generate spatially normalized intensities. For example, at each sequencing cycle, the 5% and the 95% of the intensities of the first fluorescent image and the second fluorescent image can be normalized to zero and one. If a sequencing cycle is within an indexed read, then the $95^{th}$ percentile from the last cycle of a non-indexed read can be used for normalization. Spatial normalization can reduce cycle dependent intensity variation.

FIGS. 7A-D are schematic plots showing color correction and phase correction for single light source, two-optical channel sequencing. FIG. 7A is a scatterplot of the extracted intensities or the spatially normalized intensities from the first fluorescent image versus the extracted intensities from corresponding positions in the second fluorescent image at positions ($x_i$, $y_i$) when there is no cross-talk between the first fluorescent label and the second fluorescent label. $x_i$ denotes the spatially normalized intensity of a cluster i of growing primer-polynucleotides in the second fluorescent image. $y_i$ denotes the spatially normalized intensity of the cluster i of growing primer-polynucleotides in the first fluorescent image. Because a dGTP analog includes neither the first fluorescent label nor the second fluorescent label, it has no fluorescent emission in the first fluorescent image or the second fluorescent image. Thus the population of dGTP analogs is at the position (0, 0) of the scatterplot. Because a dTTP analog includes the first fluorescent label, it has fluorescent emissions in the first fluorescent image and not the second fluorescent image. Thus the population of dTTP analogs is at the position (0, 1) of the scatterplot. Because a dCTP analog includes the second fluorescent label, it has fluorescent emissions in the second fluorescent image and not the first fluorescent image. Thus the population of dCTP analogs is at the position (1, 0)

of the scatterplot. Because a dATP analog includes the first fluorescent label and the second fluorescent label, it has fluorescent emissions in the first fluorescent image and the second fluorescent image. The population of dATP analogs is at the position (1, 1) of the scatterplot because there is no cross-talk between the first fluorescent label and the second fluorescent label.

FIG. 7B shows a schematic illustration of a scatterplot when the two fluorescent labels have overlapping emission spectra and are subject to cross-talk. Because the first fluorescent label and the second fluorescent label are subject to cross-talk, dTTP analogs have stronger emissions in the first fluorescent image and weaker emissions in the second fluorescent image. Thus, the cloud that corresponds to the fluorescent emissions from the population of dTTP analogs is at a position around (0, 1), for example (0.2, 0.8). dCTP analogs have stronger emissions in the second fluorescent image and weaker emissions in the first fluorescent image. Thus the cloud that corresponds to the fluorescent emissions from the population of dCTP analogs is at a position around (1, 0), for example (0.8, 0.2). The cloud that corresponds to the fluorescent emissions from the population of dATP analogs is at a position around (1, 1), for example, (0.9, 0.9).

To reduce or eliminate the cross-talk between the first fluorescent label and the second fluorescent label, the extracted intensities or the spatially normalized intensities can be color corrected at 545. Color correction can utilize a color matrix to condition the extracted intensities utilizing properties of the underlying distribution of intensities within each fluorescent image.

A two-channel color matrix can be a 2x2 matrix that is used to correct for the cross-talk between two channels capturing, for example a first channel and a second channel. The first channel can capture the first fluorescent images and the second fluorescent images at sequencing cycles. For example, when a cluster lights up in the first channel corresponding to the first fluorescent image, some of the emissions are also collected in the second channel corresponding to the second fluorescent image. Color correction can include using the two-channel color matrix to generate matrix corrected intensities which can reduce or eliminate the cross-talk. The color matrix can also balance any difference in overall intensity between color channels. The color matrix, M

$$\begin{pmatrix} M_{1,1} & M_{1,2} \\ M_{2,1} & M_{2,2} \end{pmatrix},$$

has cross-talk coefficients $M_{j,k}$ indicating the amount of observed intensity in channel *j* capturing the fluorescent emissions by the fluorescent label *k*. For example, $M_{1,1}$ indicates the amount of observed intensity in the first fluorescent image (i.e., channel one) capturing the fluorescent emissions by the first fluorescent label (i.e., fluorescent label one). For example, $M_{1,2}$ indicates the amount of observed intensity in the first fluorescent image (i.e., channel one) capturing the fluorescent emissions by the second fluorescent label (i.e., fluorescent label two) because of overlapping emission spectra between the first fluorescent label and the second fluorescent label.

The color matrix can be estimated based on cluster intensities collected over a configurable set of early sequencing cycles, for example sequencing cycles 1-10. This color matrix can be used for the remainder of the sequencing cycles with normalization for relative intensity that is cycle dependent.

The color matrix can be used to estimate the cross-talk between the pair of channels because they have overlapping emission spectra. In some embodiments, estimating the color matrix can include converting the plotted intensities at positions ($a_{i,\ channel\ 2}$, $a_{i,\ channel\ 1}$) into polar coordinates, where *i* denotes the cluster number, $a_{i,\ channel\ 1}$ denotes the intensity of the *i*th cluster in the first channel, and $a_{i,\ channel\ 2}$ denotes the intensity of the *i*th cluster in the second channel. Estimating the color matrix can include computing a radius-weighted histogram of angles $\theta_i$ in the range [0, 90] from the plotted intensities at position ($a_{i,\ channel\ 2}$, $a_{i,\ channel\ 1}$). For a cluster *i* with an intensity of $a_{i,\ channel\ 2}$ in the second fluorescent image and an intensity of $a_{i,\ channel\ 1}$ in the first fluorescent image, the magnitude $r_i$ can be based on the intensities $a_{i,\ channel\ 1}$ and $a_{i,\ channel\ 2}$, for example $(a_{i,\ channel\ 1}{}^2 + a_{i,\ channel\ 2}{}^2)^{1/2}$. The angle $\theta_i$ can be $\tan^{-1}(a_{i,\ channel\ 1} / a_{i,\ channel\ 2})$. FIG. 7C shows a schematic illustration of a radius-weighted histogram when the two fluorescent labels have overlapping emission spectra and are subject to cross-talk. The intensities at positions ($a_{i,\ channel\ 1}$, $a_{i,\ channel\ 2}$) in FIG. 7B can be converted into the radius-weighted angular histogram in FIG. 7C. For single light source, two channel sequencing, the radius-weighted angular histogram includes three peaks, corresponding to the clouds of dTTP analogs, dATP analogs, and dCTP analogs respectively in FIG. 7B. The center peak corresponding to the clouds of dATP analogs are at an angle of approximately 45°.

Estimating the color matrix can include identifying the two outer local maxima, $\theta_1$ and $\theta_2$, in the radius-weighted histogram. For channels that have no cross-talk, $\theta_1$ is 0° and $\theta_2$ is 90°. The cross-talk coefficient $M_{1,2}$ in the matrix can be, for example, $\tan(\theta_1)$. The cross-talk coefficient $M_{2,1}$ in the matrix can be, for example, $\tan(90-\theta_2)$. In some embodiments, if an insufficient number of clusters can be called with one of the four nucleotides, color matrix estimation may not be ideal and the identity matrix can be used instead. The diagonal elements of the matrix can be 1, and the color matrix can be

$$\begin{pmatrix} 1 & \tan(\theta_1) \\ \tan(90-\theta_2) & 1 \end{pmatrix}.$$

The color matrix can be normalized to have a determinant of 1. In some embodiments, a color matrix of an earlier sequencing cycle can be used for a subsequent sequencing cycle. The corrected intensities can be calculated by multiplying the plotted intensities in FIG. 7B by the inverse of the color matrix to generate color corrected intensities. FIG. 7D shows a schematic illustration of a scatterplot of the intensities in FIG. 7B after color correction. With corrected intensities, the individual clusters corresponding dGTP, dTTP, dCTP, and dATP can be better separated. In some embodiments, a fluorescent image can be divided into tiles, and a color matrix can be estimated for each tile. In some embodiments, a color matrix can be estimated using intensities of a number of sequencing cycles. The size and shape of the clouds of fluorescent emissions in FIGS. 7B and 7D are for illustration only. For example, the cloud that corresponds to the population of dATP analogs after color correction in FIG. 7D can be bigger than the cloud that corresponds to the population of dATP analogs before color correction in FIG. 7B.

Referring to FIG. 5, the color corrected intensities can be phase corrected at block 550. During the Sequencing by Synthesis process, each primer or extended primer in a cluster of primer-polynucleotides can extend by one base per cycle. A small proportion of strands may become out of phase with the current sequencing cycle, either falling a base behind (phasing) or running a base ahead (prephasing). For each cycle of sequencing, phasing corrections can be calculated to maximize data quality, for example, by determining a phasing matrix and applying the phasing matrix to the extracted intensities.

*Base Calling*

At block 555, the bases of nucleotides incorporated into clusters of the growing primer-nucleotides can be determined by, for example, the base caller 226. A quality score can be determined for each base called. Referring to FIG. 6, at the first sequencing cycle, because the cluster 1 has fluorescent emissions in both the first fluorescent image and the second fluorescent image, the nucleotide incorporated is a dATP analog. Because the cluster 2 has fluorescent emissions in only the second fluorescent image, the nucleotide incorporated is a dCTP analog. Because the cluster 4 has fluorescent emissions in only the first fluorescent image, the nucleotide incorporated is a dTTP analog.

At the second sequencing cycle, the nucleotides incorporated into the clusters 1-4 can be dGTP, dCTP, dTTP, and dATP respectively. After determining the existence of the cluster 3, the nucleotide incorporated into the cluster 3 during the first sequencing cycle can be dGTP which has no fluorescent emission in the first fluorescent image or the second fluorescent image. After the third sequencing cycle, the clusters 1-4 can be determined to have nucleotide sequence of AGT, CCA, GTA, and TAG respectively.

In some embodiments, base calling at block 555 can be based on the corrected intensities from block 535. The correspondence between nucleotides and the populations on the scatterplot in FIG. 7D can be defined as the follows: if a population is off in the first channel and off in the second channel, the nucleotide incorporated is a dGTP analog; if a population is off in the second channel and on in the first channel, the nucleotide incorporated is a dTTP analog; if a population is on in the second channel and off in the first channel, the nucleotide incorporated is a dCTP analog; and if a population is on in the first channel and on the second channel, the nucleotide incorporated base call is a dATP analog.

Base calling can include normalizing the corrected intenstities to (0, 1) by the $5^{th}$ and $95^{th}$ percentiles. Four Gaussian distributions, one for each of dGTP, dTTP, dCTP, and dATP can be fitted to the data of corrected and normalized intensities via an expectation maximization algorithm. The expectation maximization algorithm can determine what means and distributions best fit the data. After calculating the Gaussian distriubtions, for each population the likelihood of the population belonging to each Gaussian can be calculated. Base calling can be based on the greatest likelihood of the population belonging to a particular Gausian. For low diversity samples, the expectation maximization algorithm can be used to identify covariance matrices to avoid overfitting data. Subsampling targets can be increated to sample larger amounts of data for accuracy.

In some embodiments, the populations can be filtered by a chastity metric. A chastity metric can be, for example, D1 / (D1 + D2). D1 can be the distance to the nearest Gaussian mean, and D2 can be the distance to the next closest distance. The distance can be measured using, for example, the Mahalanobis method which can take into account the width of the distribuition along the line defined by each Gaussian centriod and the point under consideration.

At block 560, one or more quality metrics can be determined before the method ends at block 565. Sequencing quality metrics can provide important information about the accuracy of each step in this process, including library preparation, base calling, read alignment, and variant calling. Base calling accuracy, measured by the Phred quality score (Q score), can be used to assess the accuracy of a sequencing platform. It can indicate the probability that a given base is called incorrectly by the sequencer. The Q score can be $-10 \log_{10} P$, wherein P is the base calling error probability.

*Cluster Scaling*

In some embodiments, correcting intensities can include cluster scaling. Clusters can have varying brightness. For example, some clusters can be bright, and some clusters can be dim. The cluster birghtness can be caused by fragment length distribution of the sample. The varying brightness of the cluster population can have the effect of elongating the 'on' populations in the base calling scatterplot. It can be advantageous to normalize each cluster's intensity by its mean intensity in the first 10 cycles to reduce population intensity variation. For example, in the first ten cycles, for every non-guanine(G) base call, two radii can be calculated: the distance of the population intensity from the origin, and the distance of the corresponding Gaussian mean from the origin. Cluster scaling can include normalizing to the mean of the ratio of these two radii averaged over, for example, the first 10 cycles. All cluster intensities can be normalized by this scaling factor before phase correction and base calling are performed. Cluster scaling can advantageously increase throughput and decrease error rates, for example, for samples with large fragment length distributions.

Single-Light Source, Multiple-Optical Channel Sequencer

Disclosed herein are embodiments of a system or a method for determining the nucleotide sequence of polynucleotides. In one embodiment, the system includes, or is in communication with, a single light source, such as a laser or a LED light source, configured to generate light, such as light at a pre-determined wavelength. The system can include, or is in communication with, at least one detector configured to detect four substantially different fluorescent emissions off different fluorophores attached to nucleotides. The system can cause the light source to generate light onto a nucleotide. The nucleotide may be identified as a first type when a first fluorescent emission is detected by the at least one detector. The nucleotide may be identified as a second type when a second fluorescent emission is detected by the at least one detector. The nucleotide can be identified as a third type when a third fluorescent emission is detected by the at least one detector. The nucleotide can be identified as a fourth type when a fourth fluorescent emission is detected by the at least one detector. At least two of the first fluorescent emission, the second fluorescent emission, the third fluorescent emission, and the fourth fluorescent emissions may have substantially different wavelengths.

Different types of nucleotides can be attached to different fluorophores or no fluorophore. For example, a nucleotide of the first type may not be attached to a fluorophore excitable by the single light source, and the first fluorescent emission comprises no emission. In another example, a nucleotide of the first type may be attached to two different fluorophores, and the first fluorescent emission comprises emissions from the two different fluorophores.

In yet another example, the first fluorescent emission is from a first fluorophore attached to a first nucleotide of the first type, the second fluorescent emission is from a second fluorophore attached to a second nucleotide of the second type, the third fluorescent emission is from a third fluorophore attached to a third nucleotide of the third type, and the fourth fluorescent emission is from a fourth fluorophore attached to a fourth nucleotide of the fourth type. The four fluorophores may be excited using a light source. In one implementation, all four of the first fluorophore, the second fluorophore, the third fluorophore, and the fourth fluorophore are different. For example, the nucleotide sequence may be determined based on emissions by four dyes at four different wavelengths. In another implementation, three of the first fluorophore, the second fluorophore, the third fluorophore, and the fourth fluorophore are different. For example, the nucleotide sequence may be determined based on emissions by three dyes at three different wavelengths. In another implementation, two of the first fluorophore, the second fluorophore, the third fluorophore, and the fourth fluorophore are identical. For example, the nucleotide sequence may be determined based on emissions by two dyes at two different wavelengths.

Sequencing Methods

The methods described herein can be used in conjunction with a variety of nucleic acid sequencing techniques. Particularly applicable techniques are those wherein nucleic acids are attached at fixed locations in an array such that their relative positions do not change and wherein the array is repeatedly imaged. Embodiments in which images are obtained in different color channels, for example, coinciding with different labels used to distinguish one nucleotide base type from another are particularly applicable. In some embodiments, the process to determine the nucleotide sequence of a target nucleic acid can be an automated process. Preferred embodiments include sequencing-by-synthesis ("SBS") techniques.

"Sequencing-by-synthesis ("SBS") techniques" generally involve the enzymatic extension of a nascent nucleic acid strand through the iterative addition of nucleotides against a template strand. In traditional methods of SBS, a single nucleotide monomer may be provided to a target nucleotide in the presence of a polymerase in each delivery. However, in the methods described herein, more than one type of nucleotide monomer can be provided to a target nucleic acid in the presence of a polymerase in a delivery.

It is to be noted that the present disclosure may also employ the following configurations:

(1) A system for determining the nucleotide sequence of polynucleotides, comprising:

a single light source configured to stimulate emission of fluorescent light;
at least one detector configured to detect fluorescent emissions off a fluorophore attached to a nucleotide, the at least one detector being configured to detect the fluorescent emissions at a first wavelength and a second wavelength;
a processor configured to execute instructions that perform a method comprising:

generating light from the light source onto a nucleotide;
identifying the nucleotide as a first type when no fluorescent emission is detected by the at least one detector;
identifying the nucleotide as a second type when a fluorescent emission at the first wavelength of light is detected by the at least one detector;
identifying the nucleotide as a third type when a fluorescent emission at the second wavelength of light is detected by the at least one detector; and
identifying the nucleotide as a fourth type when fluorescent emissions at the first wavelength and the second wavelength of light are detected by the at least one detector.

(2) The system of (1), wherein the processor is further configured to determine the intensity of one or more of the fluorescent emissions.
(3) The system of (2), wherein the processor is further configured to determine the intensity of one or more of the fluorescent emissions by color correcting the intensity.
(4) The system of (3), wherein color correcting the intensity comprises estimating a color matrix.
(5) The system of (4), wherein estimating the color matrix comprises:

generating a radius-weighted angular histogram from a scatterplot of intensities observed in two channels; and
estimating angles of two outer local maxima $\theta_1$ and $\theta_2$ in the radius-weighted angular histogram

wherein the color matrix is $\begin{pmatrix} 1 & \tan(\theta_1) \\ \tan(90-\theta_2) & 1 \end{pmatrix}$.

(6) The system of (1), wherein the system comprises a mounting stage for a flowcell having at least one fluidic channel.
(7) The system of (1), wherein the light source is a laser, and wherein the predetermined wavelength of light generated by the laser is between 400 nm and 800 nm.
(8) The system of (1), wherein the light source is a light-emitting diode, and wherein the predetermined wavelength of light generated by the light-emitting diode is between 400 nm and 800 nm.
(9) The system of (1), wherein the at least one detector is configured to detect at least two wavelengths of light from the same fluorescent label.
(10) The system of (1), wherein the first wavelength and the second wavelength are at least 10 nm apart from one another.
(11) The system of (1), wherein the first wavelength and the second wavelength are at most 100 nm apart from one another.
(12) The system of (1), wherein the processor is further configured to identify cross-talk between the first wavelength and the second wavelength in the fluorescent emissions.
(13) A computer-implemented method for determining the nucleotide sequence of polynucleotides, comprising:

generating fluorescent light emissions using a light source onto a fluorophore attached to a nucleotide;
detecting the fluorescent light emissions off the fluorophore attached to the nucleotide at a first wavelength and a second wavelength using at least one detector; and
identifying the nucleotide, comprising

identifying the nucleotide as a first type when no fluorescent emission is detected by the at least one detector;
identifying the nucleotide as a second type when a fluorescent emission at the first wavelength of light is detected by the at least one detector;
identifying the nucleotide as a third type when a fluorescent emission at the second wavelength of light is detected by the at least one detector; and
identifying the nucleotide as a fourth type when fluorescent emissions at the first wavelength and the second wavelength of light are detected by the at least one detector.

(14) The method of (13), wherein detecting fluorescent emissions comprises color correcting the fluorescent emissions.

(15) The method of (13), wherein the light source is a laser, and wherein the predetermined wavelength of light generated by the laser is between 450 nm and 490 nm.
(16) The method of (13), wherein the light source is a light-emitting diode, and wherein the predetermined wavelength of light generated by the light-emitting diode is between 450 nm and 490 nm.
(17) The method of (13), wherein the first wavelength and the second wavelength are at least 20 nm apart from one another.
(18) The method of (13), wherein the first wavelength and the second wavelength are at most 200 nm apart from one another.
(19) The method of (13), wherein detecting fluorescent emissions comprises receiving a first fluorescent image and a second florescence image, and wherein the first fluorescent image is generated by a first fluorescent label, and wherein the second fluorescent image is generated by a second fluorescent label.
(20) The method of (19), wherein the first fluorescent label comprises Alexa 488, 3,6-Bis(ethylamino)-2,7-dimethyl-[2-carboxylato-5-(3-carboxypropyloxy)phenyl]xanthylium betaine (dye I-3), or 3,6-Bis(ethylamino)-2,7-dimethyl-[2-carboxylato-4-(3-carboxypropyloxy)phenyl]xanthylium betaine (dye I-4), and wherein the second fluorescent label comprises dye NR520LS.
(21) The method of (19), wherein the first fluorescent label comprises a Cy3 dye, and wherein the second fluorescent label comprises a Cy3-Cy5 dye pair.
(22) The method of (19), further comprising:

extracting intensities from the fluorescent images to generate extracted intensities; and
correcting the extracted intensities to generate corrected intensities, wherein correcting the extracted intensities comprise color correcting the extracted intensities, and wherein identifying the nucleotide comprises identifying the nucleotide based on the corrected intensities.

(23) The method of (22), further comprising, prior to extracting intensities from the fluorescent images:

generating a location template; and
registering locations in the location template to the fluorescent images.

(24) The method of (23), wherein correcting the extracted intensities further comprises:

spatially normalizing the extracted intensities; and
phase correcting the extracted intensities.

(25) The method of (24), wherein phase correcting the extracted intensities comprises:

determining a phasing matrix; and
applying the phasing matrix to the extracted intensities;

(26) The method of (23), wherein generating the location template comprises detecting cross-talk between the first fluorescent label and the second fluorescent label in the fluorescent images.
(27) The method of (19), wherein the first fluorescent label and the second fluorescent label are subject to cross-talk.
(28) The method of (19), wherein the first type of nucleotide is not conjugated to the first fluorescent label or the second fluorescent label, the second type of nucleotide is conjugated to the first fluorescent label, the third type of nucleotide is conjugated to the second fluorescent label, and the fourth type of nucleotide is not conjugated to the first fluorescent label and the second fluorescent label.
(29) The method of (13), wherein the first type of nucleotide is an analog of dGTP, the second type of nucleotide is an analog of dTTP, the third type of nucleotide is an analog of dCTP, and the fourth type of nucleotide trisphosphate is an analog of dATP.
(30) A system for determining the nucleotide sequence of polynucleotides, comprising:

a single light source configured to stimulate the generation of fluorescent light;
at least one detector configured to detect four substantially different fluorescent emissions off different fluorophores attached to nucleotides;
a processor configured to execute instructions that perform a method comprising:

generating light from the light source onto a nucleotide;
identifying the nucleotide as a first type when a first fluorescent emission is detected by the at least one

detector;
identifying the nucleotide as a second type when a second fluorescent emission is detected by the at least one detector;
identifying the nucleotide as a third type when a third fluorescent emission is detected by the at least one detector; and
identifying the nucleotide as a fourth type when a fourth fluorescent emission is detected by the at least one detector,

(31) The system of (30), wherein the first fluorescent emission, the second fluorescent emission, the third fluorescent emission, and the fourth fluorescent emissions have substantially different wavelengths.
(32) The system of (30), wherein the processor is further configured to determine the intensity of one or more of the fluorescent emissions.
(33) The system of (32), wherein the processor is further configured to determine the intensity of one or more of the fluorescent emissions by color correcting the intensity.
(34) The system of (33), wherein color correcting the intensity comprises estimating a color matrix.
(35) The system of (30), wherein the light source is a laser, and wherein the predetermined wavelength of light generated by the laser is between 400 nm and 800 nm.
(36) The system of (30), wherein the light source is a light-emitting diode, and wherein the predetermined wavelength of light generated by the light-emitting diode is between 400 nm and 800 nm.
(37) The system of (30), wherein a nucleotide of the first type is not attached to a fluorophore excitable by the single light source, and wherein the first fluorescent emission comprises no emission.
(38) The system of (30), wherein a nucleotide of the first type is attached to two different fluorophores, and wherein the first fluorescent emission comprises emissions from the two different fluorophores.
(39) The system of (30), wherein the first fluorescent emission is from a first fluorophore attached to a first nucleotide of the first type, wherein the second fluorescent emission is from a second fluorophore attached to a second nucleotide of the second type, wherein the third fluorescent emission is from a third fluorophore attached to a third nucleotide of the third type, and wherein the fourth fluorescent emission is from a fourth fluorophore attached to a fourth nucleotide of the fourth type.
(40) The system of (39), wherein all four of the first fluorophore, the second fluorophore, the third fluorophore, and the fourth fluorophore are different.
(41) The system of (39), wherein three of the first fluorophore, the second fluorophore, the third fluorophore, and the fourth fluorophore are different.
(42) The system of (39), wherein two of the first fluorophore, the second fluorophore, the third fluorophore, and the fourth fluorophore are identical.

Terminology

In at least some of the previously described embodiments, one or more elements used in an embodiment can interchangeably be used in another embodiment unless such a replacement is not technically feasible. It will be appreciated by those skilled in the art that various other omissions, additions and modifications may be made to the methods and structures described above without departing from the scope of the claimed subject matter. All such modifications and changes are intended to fall within the scope of the subject matter, as defined by the appended claims.
With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity.
It will be understood by those within the art that, in general, terms used herein, and especially in the appended claims (*e.g.*, bodies of the appended claims) are generally intended as "open" terms (*e.g.,* the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an" (*e.g.,* "a" and/or "an" should be interpreted to mean "at least one" or "one or more"); the same holds true for the use of definite articles used to introduce claim recitations. In addition, even if a specific number of an introduced claim recitation is explicitly recited, those skilled in the art will recognize that such recitation should

be interpreted to mean at least the recited number (*e.g.*, the bare recitation of "two recitations," without other modifiers, means at least two recitations, or two or more recitations). Furthermore, in those instances where a convention analogous to "at least one of A, B, and C, etc." is used, in general such a construction is intended in the sense one having skill in the art would understand the convention (*e.g.*, " a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (*e.g.*, " a system having at least one of A, B, or C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

In addition, where features or aspects of the disclosure are described in terms of Markush groups, those skilled in the art will recognize that the disclosure is also thereby described in terms of any individual member or subgroup of members of the Markush group.

As will be understood by one skilled in the art, for any and all purposes, such as in terms of providing a written description, all ranges disclosed herein also encompass any and all possible sub-ranges and combinations of sub-ranges thereof. Any listed range can be easily recognized as sufficiently describing and enabling the same range being broken down into at least equal halves, thirds, quarters, fifths, tenths, etc. As a non-limiting example, each range discussed herein can be readily broken down into a lower third, middle third and upper third, etc. As will also be understood by one skilled in the art all language such as "up to," "at least," "greater than," "less than," and the like include the number recited and refer to ranges which can be subsequently broken down into sub-ranges as discussed above. Finally, as will be understood by one skilled in the art, a range includes each individual member. Thus, for example, a group having 1-3 articles refers to groups having 1, 2, or 3 articles. Similarly, a group having 1-5 articles refers to groups having 1, 2, 3, 4, or 5 articles, and so forth.

While various aspects and embodiments have been disclosed herein, other aspects and embodiments will be apparent to those skilled in the art. The various aspects and embodiments disclosed herein are for purposes of illustration and are not intended to be limiting, with the true scope and spirit being indicated by the following claims.

## Claims

**1.** A system for determining the nucleotide sequence of polynucleotides, comprising:

a flowcell (114);
a single light source (120) configured to generate light at a predetermined wavelength to stimulate emission of fluorescent light;
a detector (126) coupled with a filter assembly comprising a first filter configured to transmit fluorescent emissions at a first wavelength and a second filter configured to transmit fluorescent emissions at a second wavelength, wherein the detector is configured to detect fluorescent emissions off a fluorophore attached to a nucleotide; and
a processor (202) configured to control the single light source (120) and the detector (126) and further configured to execute instructions that perform a method comprising:

generating light at the predetermined wavelength from the single light source onto a nucleotide in a polynucleotide;
detecting fluorescent light emissions from the nucleotide stimulated by the single light source (120) at the detector (126) in a first fluorescent image using the first filter and in a second fluorescent image using the second filter;
generating a location template to determine the position of the polynucleotide on the flowcell (114) using the first fluorescent image and the second fluorescent image without color correction;
extracting intensities for the position of the polynucleotide at the first wavelength from the first fluorescent image and the second wavelength from the second fluorescent image;
color correcting the extracted intensities to generate color-corrected intensities;
identifying the nucleotide in the polynucleotide associated with the determined position, based on the color-corrected intensities of fluorescent emissions stimulated by the single light source, comprising:

identifying the nucleotide as a first type when no fluorescent emission is detected in the first fluorescent image or the second fluorescent image;
identifying the nucleotide as a second type when a fluorescent emission with a color-corrected intensity

at the first wavelength of light is detected in the first fluorescent image;
identifying the nucleotide as a third type when a fluorescent emission with a color-corrected intensity at the second wavelength of light is detected in the second fluorescent image; and
identifying the nucleotide as a fourth type when fluorescent emissions with color-corrected intensities at the first wavelength and the second wavelength of light are detected in the first fluorescent image and the second fluorescent image; and

determining whether to detect more nucleotides from the polynucleotide based on (i) a quality of the fluorescent light emissions from the nucleotide or (ii) a number of bases that have been determined in the polynucleotide.

**2.** The system of claim 1, wherein color correcting the intensities comprises estimating a color matrix.

**3.** The system of claim 2, wherein color correcting the intensities further comprises:

inverting the color matrix to obtain an inverse color matrix; and
multiplying the inverse color matrix with the extracted intensities to generate the color-corrected intensities.

**4.** The system of claim 2, wherein estimating the color matrix comprises:

generating a radius-weighted angular histogram from a scatterplot of extracted intensities of a plurality of nucleic acid clusters observed in the two fluorescent images, wherein the plurality of nucleic acid clusters comprises the cluster formed of the polynucleotide; and
estimating angles of two outer local maxima $\theta_1$ and $\theta_2$ in the radius-weighted angular histogram

wherein the color matrix is $\begin{pmatrix} 1 & \tan(\theta_1) \\ \tan(90-\theta_2) & 1 \end{pmatrix}$.

**5.** The system of claim 4, wherein the radius weighted angular histogram has three maxima.

**6.** The system of any one of the preceding claims, wherein the system (100) comprises a fluidics system (104) controlled by the processor and configured to direct flows of sequencing reagents to and from the flowcell (114), wherein if a determination to detect more nucleotides from the polynucleotide is made, the method further comprises causing the fluidics system to supply, to the flowcell, a first type of nucleotide that is not conjugated to any first fluorescent label, a second type of nucleotide conjugated to a first fluorescent label, a third type of nucleotide conjugated to a second fluorescent label, and a fourth type of nucleotide conjugated to both the first fluorescent label and the second fluorescent label.

**7.** The system of any one of the preceding claims,

wherein the light source (120) is a laser, and wherein the predetermined wavelength of light generated by the laser is between 400 nm and 800 nm, or
wherein the light source (120) is a light-emitting diode, and wherein the predetermined wavelength of light generated by the light-emitting diode is between 400 nm and 800 nm

**8.** The system of any one of the preceding claims, wherein the first wavelength and the second wavelength are at least 10 nm apart from one another, and/or at most 100 nm apart from one another.

**9.** The system of any one of the preceding claims, wherein the processor (202) is further configured to identify cross-talk between the first wavelength and the second wavelength in the fluorescent emissions.

**10.** The system of any one of the preceding claims, wherein the location template is generated solely in a first sequencing by synthesis cycle.

**11.** The system of any one of the preceding claims, wherein the method further comprises:

scaling extracted intensities of a plurality of nucleic acid clusters spatially across the first fluorescent image and spatially across the second fluorescent image, prior to color correcting the extracted intensities, wherein the

plurality of nucleic acid clusters comprises the cluster formed of the polynucleotide; and/or
phase correcting the color-corrected intensities.

**12.** The system of claim 11, further comprising normalizing (i) the color-corrected intensities or (ii) the phase- and color-corrected intensities, wherein identifying the nucleotide is based on the normalized intensities.

**13.** The system of claim 12, wherein identifying the nucleotide comprises fitting four Gaussian distributions, one for each nucleotide type, to the normalized intensities via an expectation-maximization algorithm, and determining the nucleotide based on the greatest likelihood derived from the fitted Gaussian distributions.

**14.** The system of any one of the preceding claims, wherein the quality of the fluorescent light emissions from the nucleotide comprises a Phred quality score and/or relates to the probability that the nucleotide is called incorrectly by the system.

**15.** The system of claim 6, wherein:

the first fluorescent label comprises Alexa 488, 3,6-Bis(ethylamino)-2,7-dimethyl-[2-carboxylato-5-(3-carboxypropyloxy)phenyl]xanthylium betaine (dye I-3), or 3,6-Bis(ethylamino)-2,7-dimethyl-[2-carboxylato-4-(3-carboxypropyloxy)phenyl]xanthylium betaine (dye I-4), and the second fluorescent label comprises dye NR520LS, or
the first fluorescent label comprises a Cy3 dye, and the second fluorescent label comprises a Cy3-Cy5 dye pair.

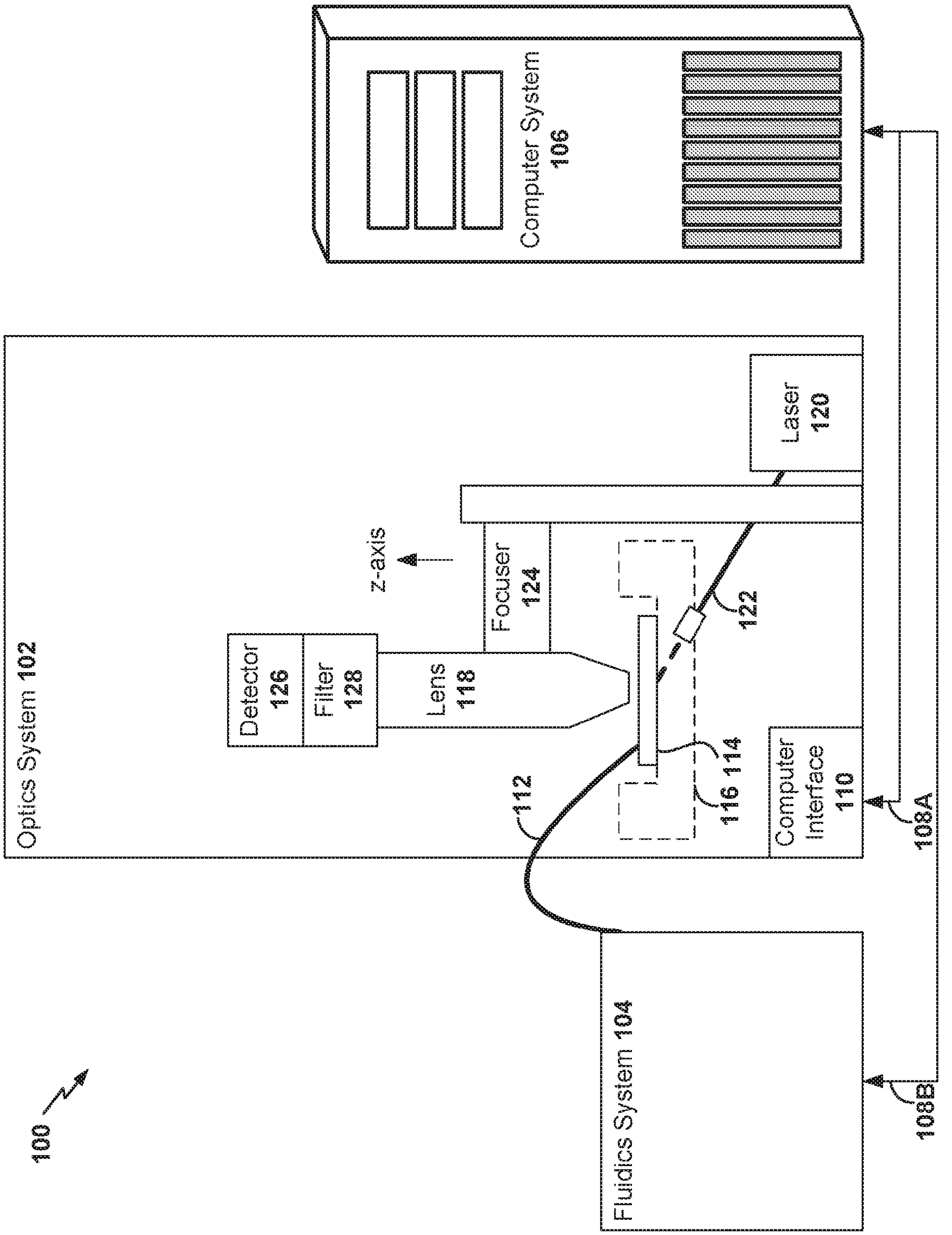

100
Optics System 102
Fluidics System 104
Computer System 106
108A
108B
Computer Interface 110
112
114
116
Lens 118
Laser 120
122
Focuser 124
Detector 126
Filter 128
z-axis


FIG. 1

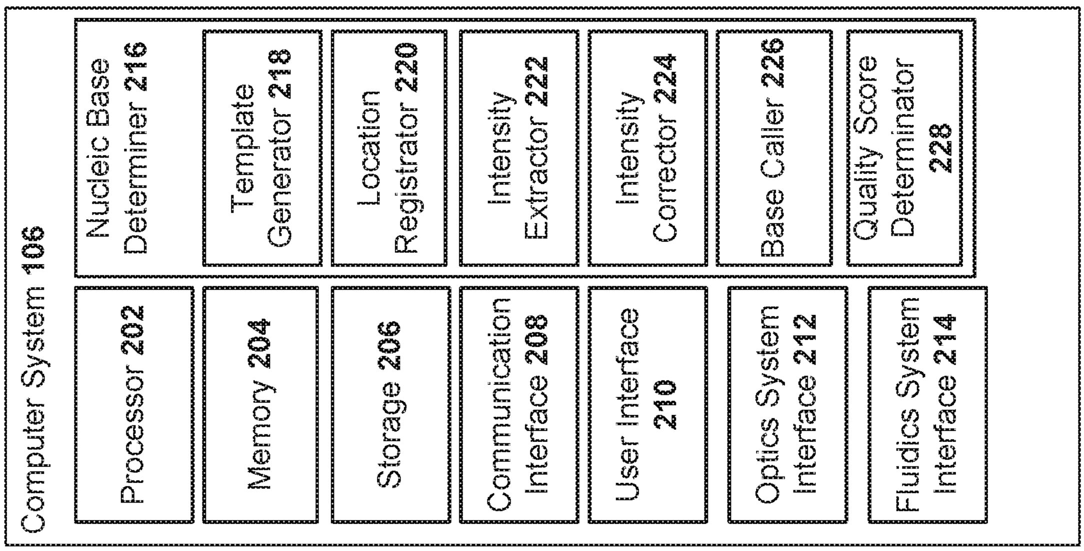
Computer System 106
Processor 202
Memory 204
Storage 206
Communication Interface 208
User Interface 210
Optics System Interface 212
Fluidics System Interface 214
Nucleic Base Determiner 216
Template Generator 218
Location Registrator 220
Intensity Extractor 222
Intensity Corrector 224
Base Caller 226
Quality Score Determinator 228

FIG. 2

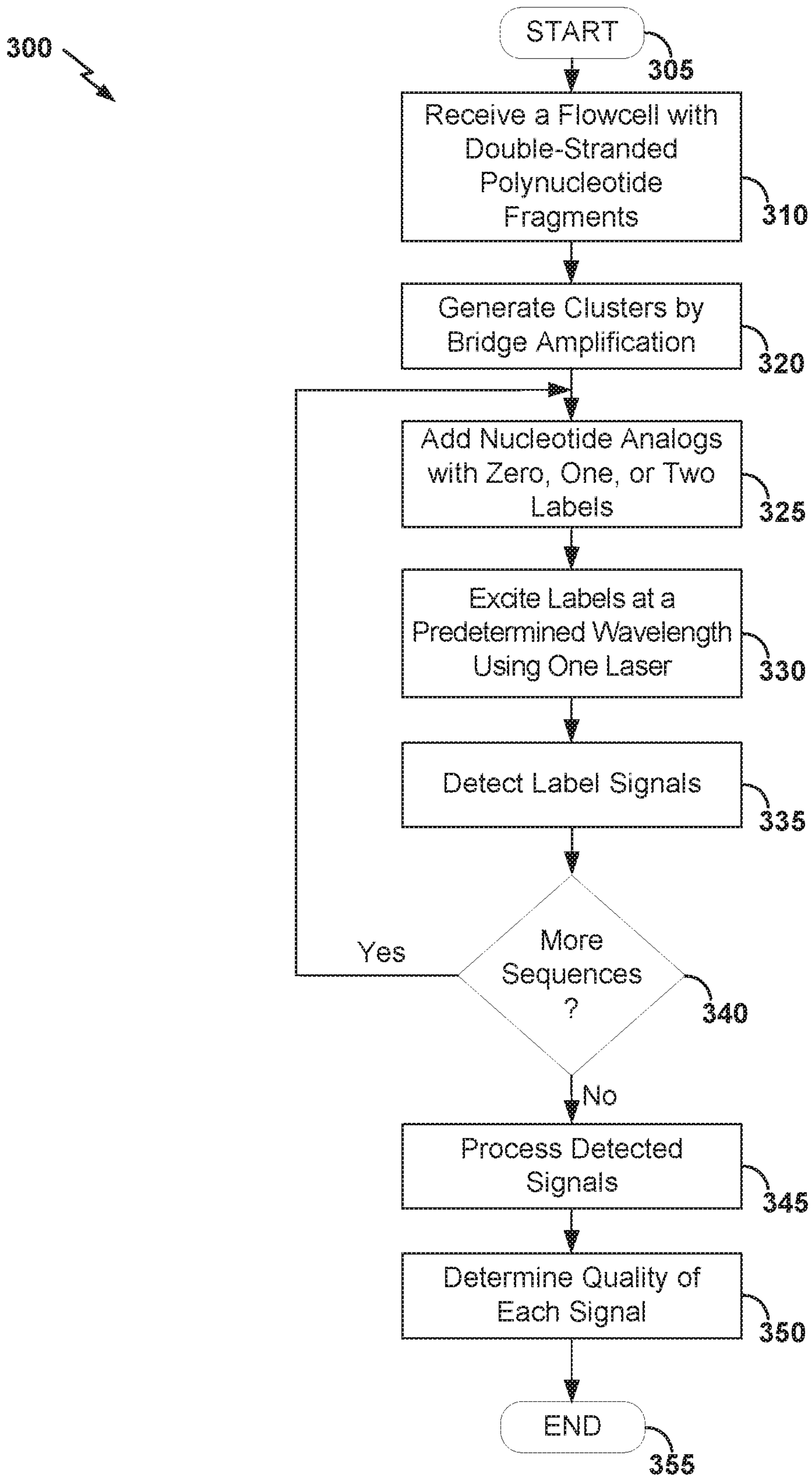
300
START
305
Receive a Flowcell with Double-Stranded Polynucleotide Fragments
310
Generate Clusters by Bridge Amplification
320
Add Nucleotide Analogs with Zero, One, or Two Labels
325
Excite Labels at a Predetermined Wavelength Using One Laser
330
Detect Label Signals
335
More Sequences ?
340
Yes
No
Process Detected Signals
345
Determine Quality of Each Signal
350
END
355

FIG. 3

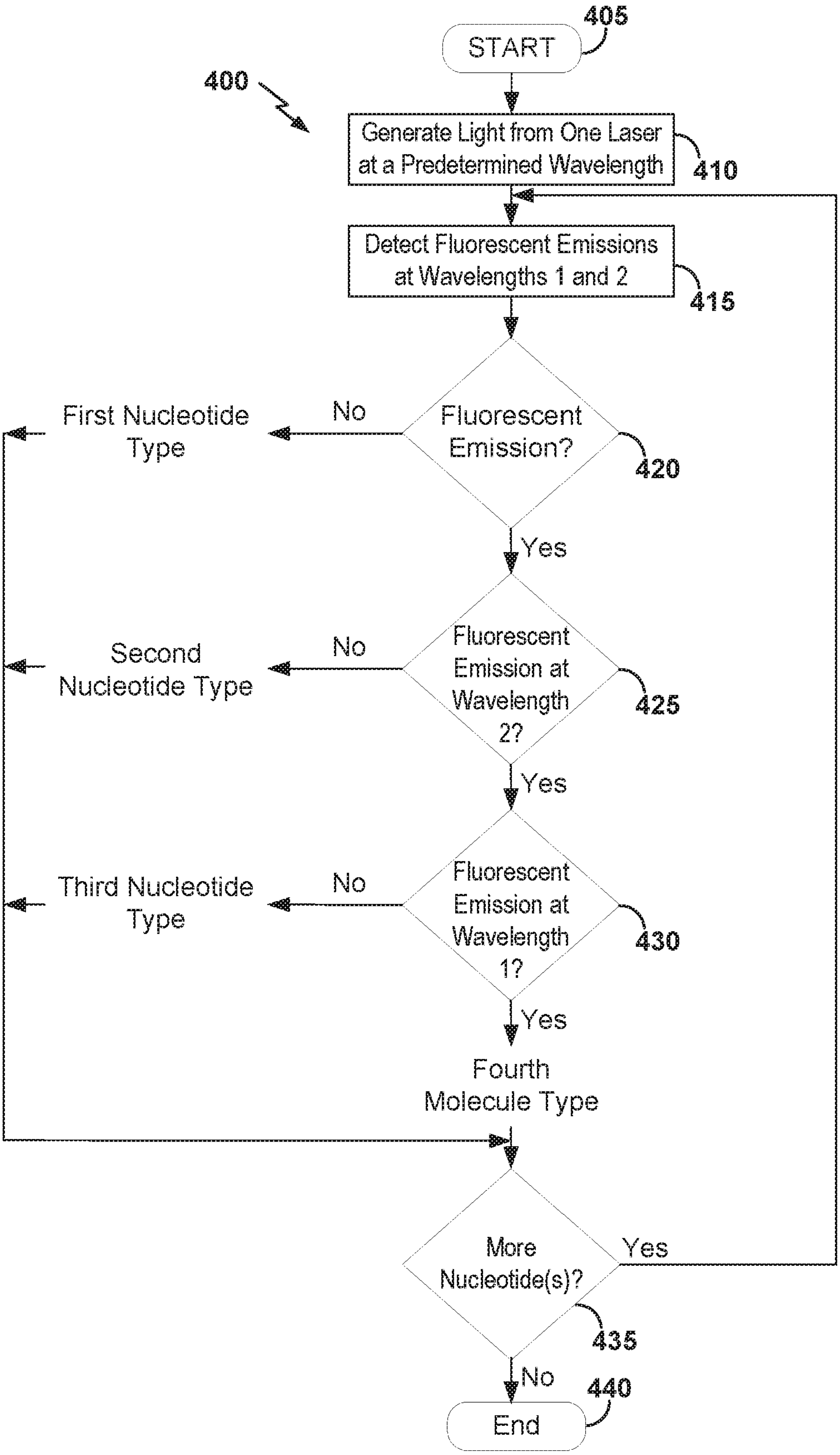
400
START
405
Generate Light from One Laser at a Predetermined Wavelength
410
Detect Fluorescent Emissions at Wavelengths 1 and 2
415
Fluorescent Emission?
420
No
First Nucleotide Type
Yes
Fluorescent Emission at Wavelength 2?
425
No
Second Nucleotide Type
Yes
Fluorescent Emission at Wavelength 1?
430
No
Third Nucleotide Type
Yes
Fourth Molecule Type
More Nucleotide(s)?
435
Yes
No
End
440

FIG. 4

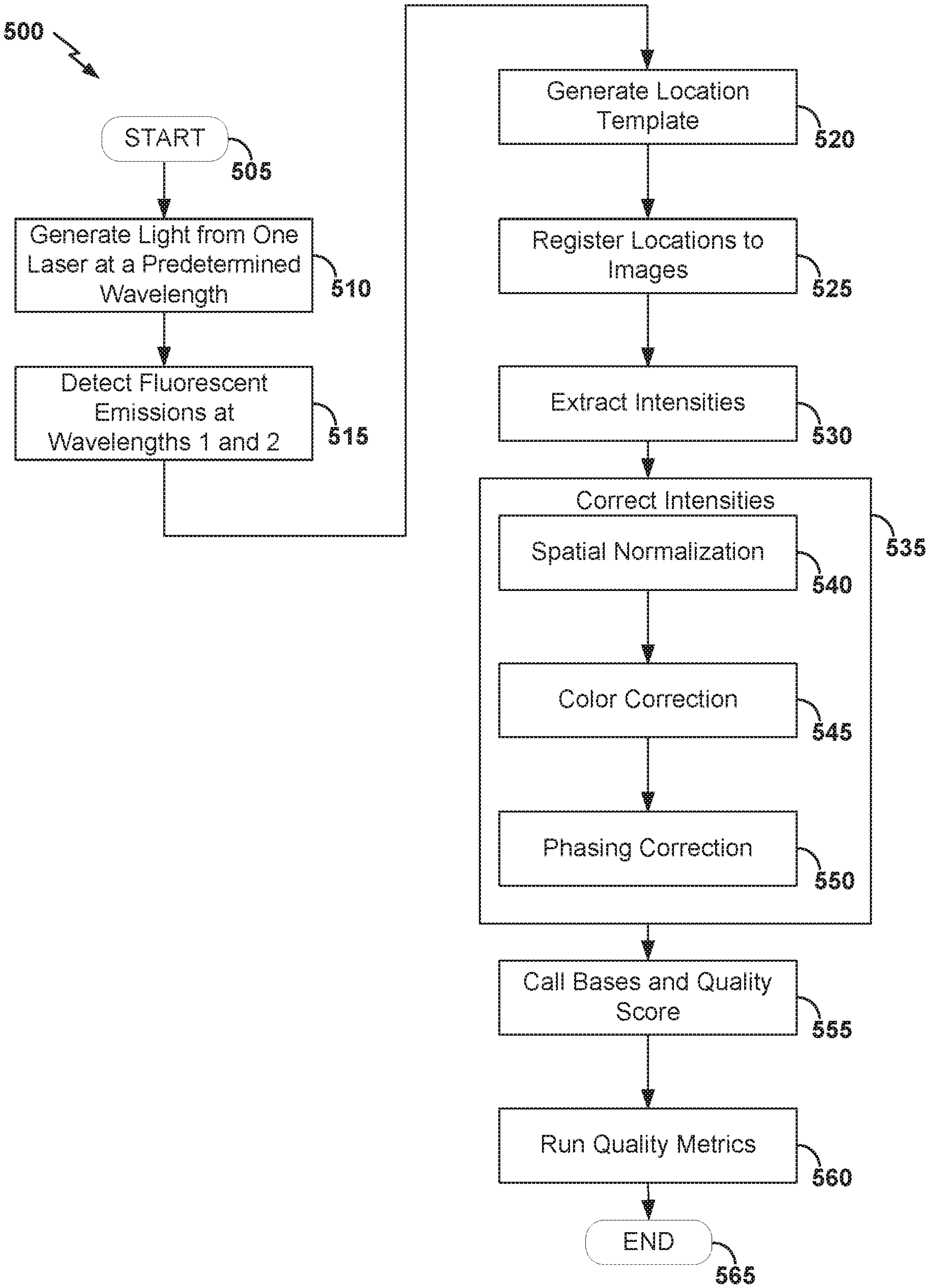
500
START
505
Generate Light from One Laser at a Predetermined Wavelength
510
Detect Fluorescent Emissions at Wavelengths 1 and 2
515
Generate Location Template
520
Register Locations to Images
525
Extract Intensities
530
Correct Intensities
535
Spatial Normalization
540
Color Correction
545
Phasing Correction
550
Call Bases and Quality Score
555
Run Quality Metrics
560
END
565

*FIG. 5*

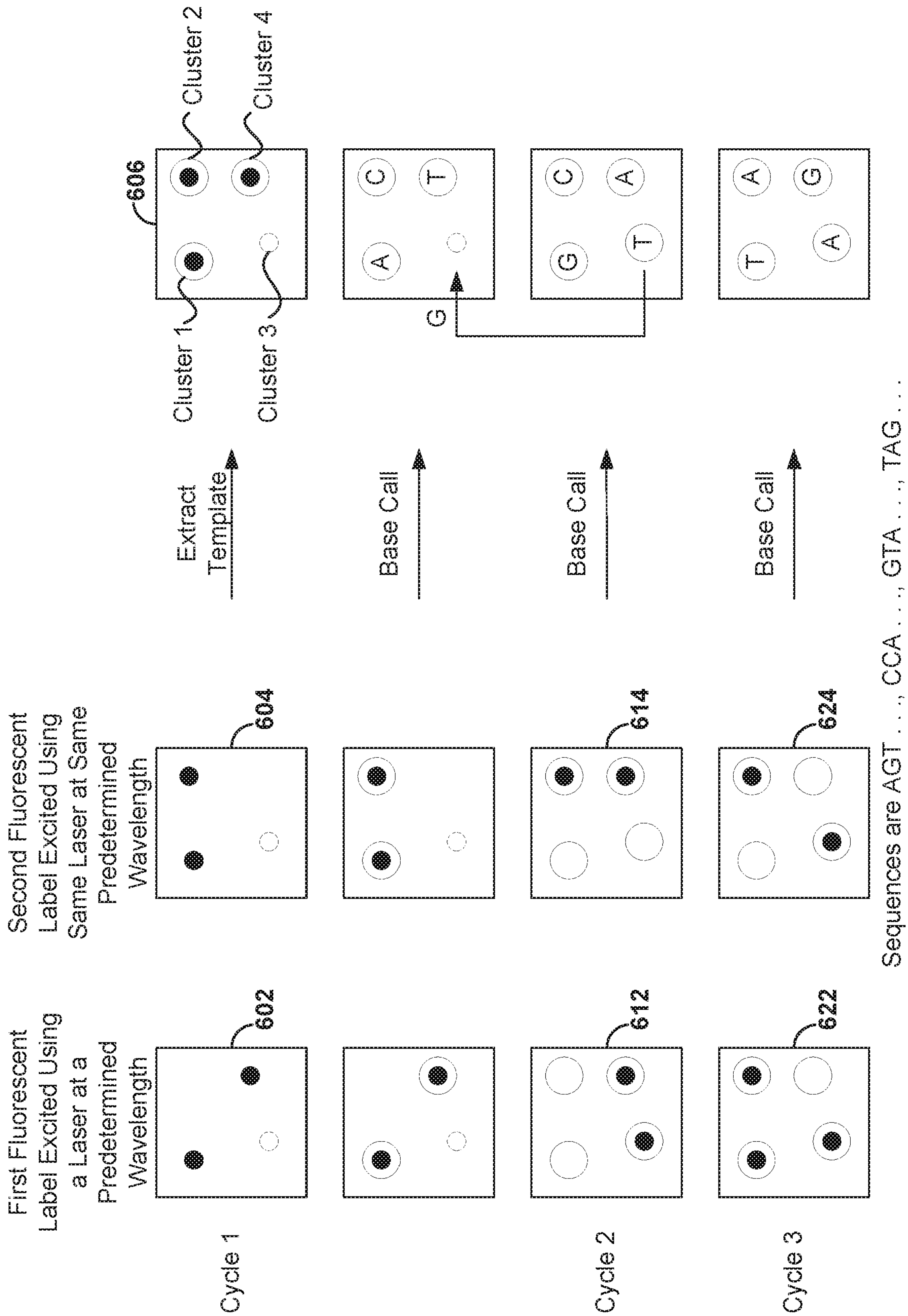
First Fluorescent Label Excited Using a Laser at a Predetermined Wavelength
Second Fluorescent Label Excited Using Same Laser at Same Predetermined Wavelength
Cycle 1
Cycle 2
Cycle 3
602
604
606
612
614
622
624
Extract Template
Base Call
Base Call
Base Call
Cluster 1
Cluster 2
Cluster 3
Cluster 4
A
C
T
G
C
T
A
G
T
A
A
G
Sequences are AGT . . ., CCA . . ., GTA . . ., TAG . . .

FIG. 6

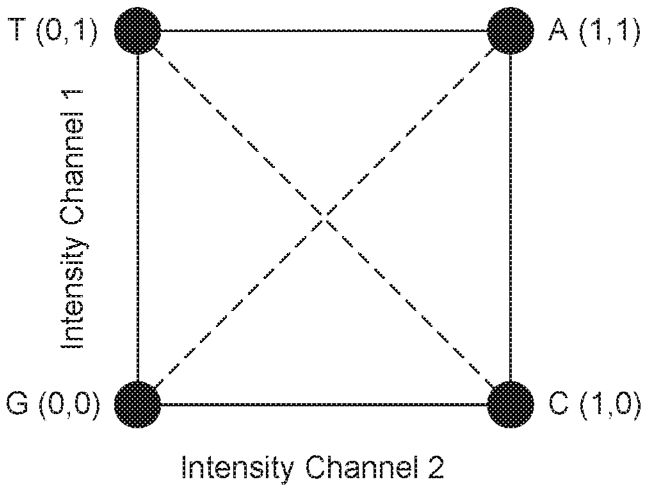


FIG. 7A

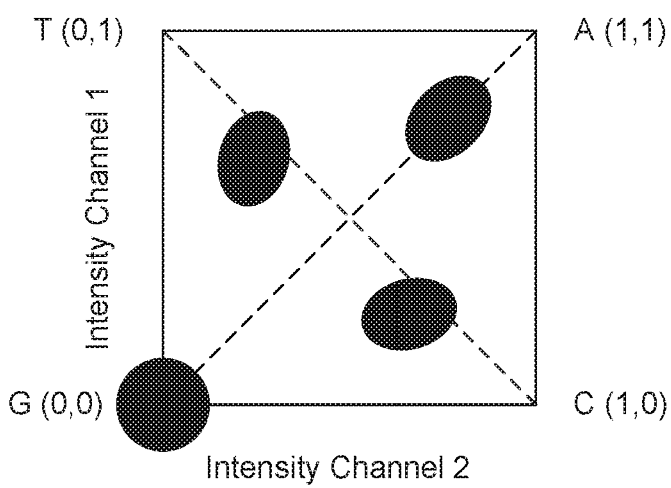


FIG. 7B

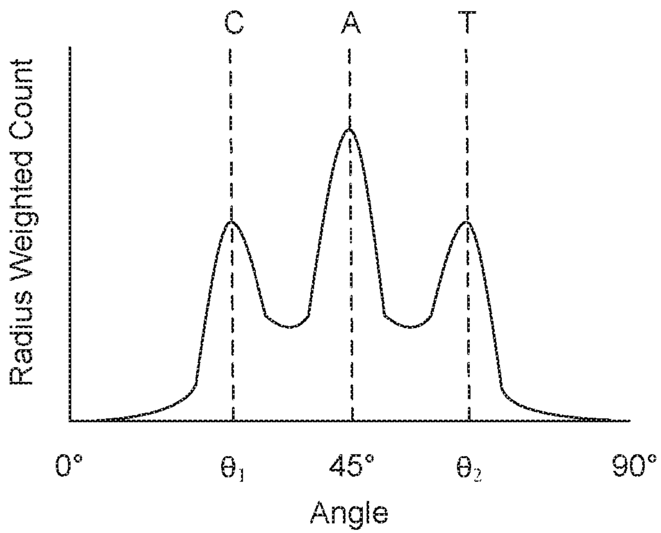


FIG. 7C

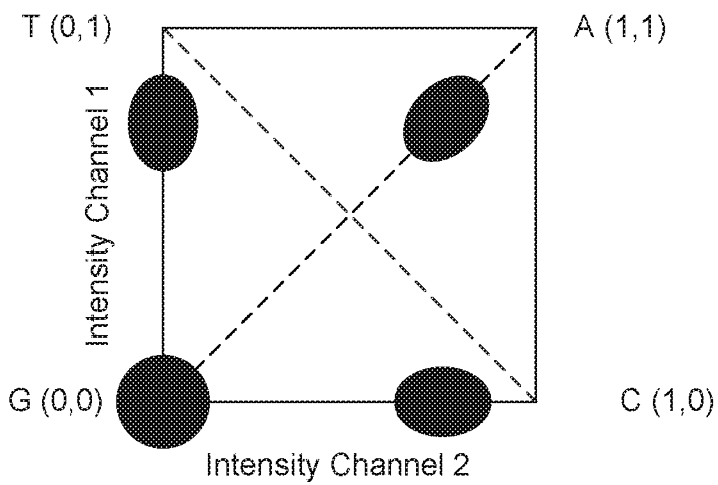


FIG. 7D

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62468242
- US 8754244 B
- GB 2016051474 W
- US 8965076 B
- US 530299


### Non-patent literature cited in the description


- **SINGLETON et al.** Dictionary of Microbiology and Molecular Biology. J. Wiley & Sons, 1994
- **SAMBROOK et al.** Molecular Cloning, A Laboratory Manual. Cold Spring Harbor Press, 1989